# EUROPEAN PATENT APPLICATION

(11) **EP 4 215 208 A1**
(43) Date of publication of application: **26.07.2023**
(21) Application number: 21868764.8
(22) Date of filing: 22.09.2021
(51) Int. Cl.: A61K 39/395, C07K 16/32, A61P 35/00, A61P 37/02

(54) **PHARMACEUTICAL COMBINATION AND USE THEREOF**

(30) Priority: 21.09.2020 CN 202010996664
(71) Applicant: Shanghai Bao Pharmaceuticals Co., Ltd., Shanghai 201908 (CN)
(72) Inventor: LIU, Yanjun, Shanghai 201908 (CN); WANG, Zheng, Shanghai 201908 (CN)
(74) Representative: Zwicker, Jörk
(86) International application number: PCT/CN2021/119677
(87) International publication number: WO 2022/057942

(57) **Abstract**

Provided are a pharmaceutical combination and use thereof. The pharmaceutical combination is used for preparing a medicament for treating hyperglobulinemia, and comprises an immunoglobulin degradation enzyme and an Fc-containing protein. The pharmaceutical combination uses the immunoglobulin degradation enzyme and the Fc agents in combination in hyperglobulinemia, reduces the influence of globulin on treatment, enables the Fc agents to have better effects, and also reduces the clinical dose of some Fc agents. On one hand, side effects of a therapeutic agent can be reduced, and on the other hand, the economic burden of medication can also be reduced.

## Description

### Technical Field

The present invention relates to the field of biomedicine, in particular to a pharmaceutical combination and use thereof, specifically use thereof in the preparation of a medicament for treating a disease with an abnormally elevated level of blood IgG.

### Background Art

Hyperglobulinemia is characterized by a higher than normal level of immunoglobulins in a patient. The immunoglobulins include IgA, IgD, IgE, IgM, and IgG, among which excessive γ immunoglobulin IgG is the most common. M protein, a large amount of abnormal immunoglobulins produced by monoclonal malignant proliferation from plasma cells or B lymphocytes, is essentially an immunoglobulin or a fragment thereof and is also called monoclonal protein, monoclonal immunoglobulin, myeloma protein, or M-spike.

It is termed "M protein" because it is commonly found in multiple myeloma, macroglobulinemia, and malignant lymphoma, all of which are diseases starting with the letter M. Positive M protein can be seen in malignant monoclonal gammaglobulinemia, such as multiple myeloma, heavy chain disease, malignant lymphoma, chronic lymphocytic leukemia, and macroglobulinemia; secondary monoclonal gammaglobulinemia, such as non-lymphoreticular tumors, monocytic leukemia, and cryoglobulinemia; and benign M proteinemia, and the incidence thereof in elderly people aged 70 or older is approximately 3%.

Multiple myeloma is a hematologic tumor caused by plasma cell canceration in bone marrow. Abnormal plasma cells gather in bone marrow and produce tumors in bones at multiple sites of the body. Not only do these cells fail to function normally, but they also cause bone marrow to fail to produce healthy blood cells. Abnormal proliferation of bone marrow plasma cells is accompanied by excessive production of non-functional monoclonal immunoglobulins or light chains (M protein). The level of such abnormal immunoglobulins can be as high as 30 g/L or higher in most patients (the level of IgG in the blood of normal people is about 10 g/L), and M protein in about 50% of the patients is of IgG subtype. At present, antibodies for treating multiple myeloma include daratumumab and isatuximab. The inventors have unexpectedly found that by eliminating pathological immunoglobulins in advance with an immunoglobulin degrading enzyme, the therapeutic effect of the monoclonal antibody can be enhanced while the therapeutic dose of the antibody is reduced.

### Summary of the Invention

In view of the shortcomings in the prior art, the present invention aims to apply an immunoglobulin degrading enzyme in combination with an Fc agent to hyperglobulinemia to reduce the influence of globulins on therapy, so that the Fc agent can function better; moreover, the clinical dosage of some Fc agents can also be reduced, which can reduce the side effects of the therapeutic agents on the one hand and also the economic burden of medication on the other hand.

The object of the present invention will be achieved by the following technical solutions:

### 1. Pharmaceutical combination

In a first aspect, a pharmaceutical combination is provided, which can be used for the preparation of a medicament for treating hyperglobulinemia, wherein the pharmaceutical composition comprises: 1) an immunoglobulin degrading enzyme, and 2) an Fc-containing protein. The pharmaceutical combination allows for separate administration of the degrading enzyme and the immunoglobulin. Preferably, the pharmaceutical combination comprises therapeutically effective amounts of the immunoglobulin degrading enzyme and the immunoglobulin. Preferably, the pharmaceutical combination is a pharmaceutical composition and further comprises a pharmaceutically acceptable carrier or diluent. It is a lyophilized powder or liquid.

### 1.1 Immunoglobulin degrading enzyme

The term "immunoglobulin degrading enzyme" used herein preferably refers to a protease drug or agent that reduces the blood immunoglobulin level to 60% or less of the original level, and the amino acid sequence thereof is usually derived from bacteria or comprises some sequences derived from bacteria. Preferably, the drug or agent reduces the blood immunoglobulin to at most 60%, at most 50%, at most 40%, at most 30%, at most 20%, at most 10%, or at most 0% of the original level. More preferably, the drug or agent reduces blood immunoglobulins to at most 20%, at most 10%, or at most 0% of the original level.

The agent is usually a protein with IgG cysteine protease activity, which preferably digests the hinge region of the immunoglobulin molecule. An example of this protein is IdeE (an immunoglobulin G degrading enzyme derived from *Streptococcus pyogenes*)*.* IdeE, a streptococcus protease with unique specificity, digests immunoglobulin G (IgG) antibodies, but does not digest any other substrates (including IgA, IgD, IgE, and IgM). IdeE digests human IgG into F(ab')₂ fragment and Fc fragment at a specific site at the COOH end of the hinge region. The mature sequence of IdeE is as set forth in SEQ ID NO: 5. The agent may be a protein containing or consisting of the amino acid sequence of SEQ ID NO: 5, or may be a homologue thereof from alternative bacteria.

Preferably, in the pharmaceutical combination as described above, the immunoglobulin degrading enzyme is an IgG degrading enzyme selected from an IgG cysteine protease derived from *Streptococcus pyogenes* or a variant or fragment thereof or a human-derived MMP protease or a variant or fragment thereof, wherein the variants or fragments thereof maintain IgG-digesting activity; and preferably, the IgG degrading enzyme is selected from IdeS, MAC2, IdeZ, IdeZ2, IdeE, IdeE2, IdeP, and MMPs.

The variant of IdeE protein may comprise or consist of an amino acid sequence which may have 1, 2, 3, 4, 5, 10, 20, 30 or more amino acid substitutions, insertions or deletions relative to the amino acid sequence of SEQ ID NO: 5, provided that the variant has IgG cysteine protease activity. All amino acid substitutions are preferably conservative. Conservative substitution replaces an amino acid with another amino acid with a similar chemical structure, similar chemical properties or a similar side chain volume. The introduced amino acid can be similar to the replaced amino acid in polarity, hydrophilicity, hydrophobicity, alkalinity, acidity, neutrality or charge. Alternatively, conservative substitution can introduce another aromatic or aliphatic amino acid at the position of an originally existing aromatic or aliphatic amino acid. Conservative amino acid changes are well known in the art.

As for the variant of IdeE protein, the mutant is obtained by replacement at least one or more of positions 8, 10, 24, 59, 97, and 280 of the amino acid sequence of the immunoglobulin degrading enzyme IdeE; and the function of the mutant includes at least the function of the immunoglobulin degrading enzyme IdeE.

The expression "includes at least" the function of the immunoglobulin degrading enzyme IdeE means that the mutant can further contain other functions on the basis of retaining the immunoglobulin degrading enzyme IdeE, or the mutant is more effective than IdeS in cleaving human IgG, or the mutant is as effective as IdeS in cleaving human IgG.

Alternatively, the immunoglobulin degrading enzyme may be a protein comprising or consisting of a fragment of SEQ ID NO: 1, SEQ ID NO: 2 or SEQ ID NO: 3 and has IgG cysteine protease activity, preferably wherein the length of the fragment is 100 to 300 amino acids, 150 to 300 amino acids, or 200 to 300 amino acids. The fragment can be generated by deleting one or more amino acid residues of the amino acid sequence of SEQ ID NO: 1, SEQ ID NO: 2, or SEQ ID NO: 3. At most 1, 2, 3, 4, 5, 10, 20, 30, 40, or 50 residues or more residues can be deleted. The deleted residues may be continuous with each other.

Preferably, the IgG degrading enzyme comprises an amino acid sequence as shown in Table 1, or a variant thereof with the same function, or a protein or a fragment thereof consisting of the amino acid sequence. In one embodiment, the IgG degrading enzyme comprises an amino acid sequence as shown in Table 1, and may also comprise: 1) the amino acid sequences of SEQ ID NO: 1-42, 59, 60, and 61-95; 2) variants thereof, which have at least 50% identity to the amino acid sequences of SEQ ID NO: 1-42, 59, 60, and 61-95 and have IgG degrading enzyme; or fragments of 1) and 2) with IgG degrading enzyme activity.

**Table 1. Sequences of immunoglobulin degrading enzymes**

| SEQ ID NO: | Sequence |
|---|---|
| 1 | IdeS, NCBI Reference Sequence NO. WP_010922160.1 |
| | DSFSANQEIRYSEVTPYHVTSVWTKGVTPPANFTQGEDVFHAPYVANQGWYDITKT FNGKDDLLCGAATAGNMLHWWFDQNKDQIKRYLEEHPEKQKINFNGEQMFDVKE AIDTKNHQLDSKLFEYFKEKAFPYLSTKHLGVFPDHVIDMFINGYRLSLTNHGPTPV |
| | KEGSKDPRGGIFDAVFTRGDQSKLLTSRHDFKEKNLKEISDLIKKELTEGKALGLSHT YANVRINHVINLWGADFDSNGNLKAIYVTDSDSNASIGMKKYFVGVNSAGKVAISA KEIKEDNIGAQVLGLFTLSTGQDSWNQTN |
| 2 | IdeZ, NCBI Reference Sequence NO. WP_014622780.1 |
| | DDYQRNATEAYAKEVPHQITSVWTKGVTPLTPEQFRYNNEDVIHAPYLAHQGWYDI TKAFDGKDNLLCGAATAGNMLHWWFDQNKTEIEAYLSKHPEKQKIIFNNQELFDLK AAIDTKDSQTNSQLFNYFRDKAFPNLSARQLGVMPDLVLDMFINGYYLNVFKTQST DVNRPYQDKDKRGGIFDAVFTRGDQTTLLTARHDLKNKGLNDISTIIKQELTEGRAL ALSHTYANVSISHVINLWGADFNAEGNLEAIYVTDSDANASIGMKKYFVGINAHGH VAISAKKIEGENIGAQVLGLFTLSSGKDIWQKLS |
| 3 | IdeZ, ABH04315.1 IgG endopeptidase *[Streptococcus equi* subsp. *zooepidemicus*] |
| | DDYQRNAAEVYAKEVPHQITSVWTKGVTPLTPEQFRYNNEDVIHAPYLAHQGWYD ITKVFDGKDNLLCGAATAGNMLHWWFDQNKTEIEAYLSKHPEKQKIIFNNQELFDL KAAIDTKDSQTNSQLFNYFRDKAFPNLSARQLGVMPDLVLDMFINGYYLNVFKTQS TDVNRPYQDKDKRGGIFDAVFTRGDQTTLLTARHDLKNKGLNDISTIIKQELTEGRA LALSHTYANVSISHVINLWGADFNAEGNLEAIYVTDSDANASIGMKKYFVGINAHG HVAISAKKIEGENIGAQVLGLFTLSSGKDIWQKLS |
| 4 | IdeZ2, ACU12336.1 IgG endopeptidase IdeZ2 *[Streptococcus equi* subsp. *zooepidemicus*] |
| | MMKKQSFTHSRKPKFGMRKLSIGLASCMLGMMFLTTSHVSGEVVEVWPYGQDPND KIEVLSQSEYSEYLQRLHDVEDFQAEKKKEGVVRTQWLEGVNVTDHDFRKITDGGS VYYATPLLNDRGYYDINKNFNQDSDKCAAAVAVNMFHYWLDRNKDNVAKFLSQS PDHGFVEGEPTFNLVDFQYTYASPYEEGGYRDNSKLFDFISKAFNKPLWANKLLDA YINGYGYIDRYVKNTPHSGQNNSKFNFFKKVFDGKLLTDIQQIFDYYTLSSELREALD TGKAIGLAYGPGDLRRSLGHIISVWGADINEDGNVVAIYVTDSDDKKLTIGNKKDRI GLKRYKLYSDNVGRARLTAYATENQQTGGEVRGIETLDMATQDWADYFSRTDEAE Q |
| 5 | IdeE, ABF57910.1 IgG endopeptidase *[Streptococcus equi* subsp. *equi*] |
| | DDYQRNATEAYAKEVPHQITSVWTKGVTPLTPEQFRYNNEDVIHAPYLAHQGWYDI TKAFDGKDNLLCGAATAGNMLHWWFDQNKTEIEAYLSKHPEKQKIIFNNQELFDLK AAIDTKDSQTNSQLFNYFRDKAFPNLSARQLGVMPDLVLDMFINGYYLNVFKTQST DVNRPYQDKDKRGGIFDAVFTRGDQTTLLTARHDLKNKGLNDISTIIKQELTEGRAL ALSHTYANVSISHVINLWGADFNAEGNLEAIYVTDSDANASIGMKKYFVGINAHRH VAISAKKIEGENIGAQVLGLFTLSSGKDIWQKLS |
| 6 | IdeE2, ACU12335.1 IgG endopeptidase IdeE2 *[Streptococcus equi* subsp. *equi*] |
| | EVVEVWPNGQNPNGKIEILSQTEHSEHLQKLRDIEDFQAQKQADHVRYTKWLDGVT VDEHEFRKIKEYDTEYYVTPLLSGKGYYDINKDFNQDSDKCAAAVAANMFHYWFD RNRDSINRFLSQSPGENGVIKLENEKTIEVSKFLETYRSDGDYLDKSPFFDLISNSFKG PVWANKLLDAYINGYGYIHKFAKNTPHSKNNNSKFNFFKKVFDGNLLTDIHQIFDY NTFSDKLSEALYTGKAIGLAYGPGDLRRSLGHIISVWGADLDDQNRVVAIYVTDSDD |
| | KKLTIGNERVGLKRYKVSSDDQGRARLTTRDKDNTGGEIRSIETLDMGTQEWADYF NKTEK |
| 7 | IdeP, AJD79088.1 immunglobulin degrading enzyme [*Streptococcus phocae*] |
| | DNRSASTISVSIDSNQNETNLVTAENVQTANLSDPDTKVNTVWTAGMSELDTKQFTP HPDGFLHYPYQSNQGWYNITKSFNGRDNLLCAAATAGNMLHWWFDQNKEQIDRY LKEHPEKANISFRGENFLSIKELINTKGKQKDSALFTYFQEKAFPDISRRNTGLIVDRV LDMFINGYYLSINDLSETNTSNTRKDNRGGVFDHVFGRGDSHKKLTSRYELQDQSIE EIGQLIRRELEAGKALAISYTYASIGMAHVVNLWGADFDQAGQLVAIYVADSDNST DVGMKKYYVGKNKEGHVAISAQKVEGDVLGSRLLRLGSLSSGKDMWPAQY |
| 8 | CN107532156A pCART152 |
| | DSFSANQEIRYSEVTPYHVTSVWTKGVTPPANFTQGEDVFHAPYVANQGWYDITKT FNGKDDLLCGAATAGNMLHWWFDQNKDQIKRYLEEHPEKQKINFRGEQMFDVKE AIDTKNHQLDSKLFEYFKEKAFPYLSTKHLGVFPDHVIDMFINGYRLSLTNHGPTPV KEGSKDPRGGIFDAVFTRGDQSKLLTSRHDFKEKNLKEISDLIKKELTEGKALGLSHT YANVRINHVINLWGADFDSNGNLKAIYVTDSDSNASIGMKKYFVGVNSAGKVAISA KEIKEDNIGAQVLGLFTLSTGQDSWNQTN |
| 9 | CN107532156A pCART183 |
| | DSFSANQEIRYSEVTPYHVTSVWTKGVTPPANFTQGEDVFHAPYVANQGWYDITKT FNGKDDLLCGAATAGNMLHWWFDQNKDQIKRYLEEHPEKQKINFKGEQMFDVKE AIDTKNHQLDSKLFEYFKEKAFPYLSTKHLGVFPDHVIDMFINGYRLSLTNHGPTPV KRGSKDPRGGIFDAVFTRGNQSKLLTSRHDFKEKNLKEISDLIKKELTEGKALGLSHT YANVRINHVINLWGADFDSNGNLKAIYVTDSDSNASIGMKKYFVGVNSAGKVAISA KEIKEDNIGAQVLGLFTLSTGQDSWNQTN |
| 10 | CN107532156A pCART184 |
| | DSFSANQEIRYSEVTPYHVTSVWTKGVTPPANFTQGEDVFHAPYVANQGWYDITKT FNGKDDLLCGAATAGNMLHWWFDQNKDQIKRYLEEHPEKQKINFRGEQMFDVKE AIDTKNHQLDSKLFEYFKEKAFPYLSTKHLGVFPDHVIDMFINGYRLSLTNHGPTPV KKGSKDPRGGIFDAVFTRGNQSKLLTSRHDFKEKNLKEISDLIKKELTEGKALGLSHT YANVRINHVINLWGADFDSNGNLKAIYVTDSDSNASIGMKKYFVGVNKAGKVAISA KEIKEDNIGAQVLGLFTLSTGQDSWNQTN |
| 11 | CN107532156A pCART185 |
| | DSFSANQEIRYSEVTPYHVTSVWTKGVTPPANFTQGEDVFHAPYVANQGWYDITKT FNGKDDLLCGAATAGNMLHWWFDQNKDQIKRYLREHPEKQKINFNGEQMFDVKE AIDTKNHQLDSKLFEYFKEKAFPYLSTKHLGVFPDHVIDMFINGYRLSLTNHGPTPV KEGSKDPRGGIFDAVFTRGNQSKLLTSRHDFKEKNLKEISDLIKKELDEGKALGLSHT YANVRINHVINLWGADFDSNGNLKAIYVTDSDSNASIGMKKYFVGVNSAGKVAISA KEIKEDNIGAQVLGLFTLSTGQDSWNQTN |
| 12 | CN107532156A pCART186 |
| | DSFSANQEIRYSEVTPYHVTSVWTKGVTPPANFTQGEDVFHAPYVANQGWYDITKT |
| | FNGKDDLLCGAATAGNMLHWWFDQNKDQIKRYLKEHPEKQKINFNGEQMFDVKE AIRTKNHQLDSKLFEYFKEKAFPYLSTKHLGVFPDHVIDMFINGYRLSLTNHGPTPVK EGSKDPRGGIFDAVFTRGNQSKLLTSRHDFKEKNLKEISDLIKKELEEGKALGLSHTY ANVRINHVINLWGADFDSNGNLKAIYVTDSDSNASIGMKKYFVGVNKAGKVAISAK EIKEDNIGAQVLGLFTLSTGQDSWNQTN |
| 13 | CN107532156A pCART187 |
| | DSFSANQEIRYSEVTPYHVTSVWTKGVTPPANFTQGEDVFHAPYVANQGWYDITKT FNGKDDLLCGAATAGNMLHWWFDQNKDQIERYLEEHPEKQKINFNGEQMFDVKE AIDTKNHQLDSKLFEYFKEKAFPYLSTKHLGVFPDHVIDMFINGYRLSLTNHGPTPV KEGSKDPRGGIFDAVFTRGNQSKLLTSRHDFKEKNLKEISDLIKEELTKGKALGLSHT YANVRINHVINLWGADFDSNGNLKAIYVTDSDSNASIGMKKYFVGVNSAGKVAISA KEIKEKNIGAQVLGLFTLSTGQKSWNQTN |
| 14 | CN107532156A pCART188 |
| | DSFSANQEIRYSEVTPYHVTSVWTKGVTPPANFTQGEDVFHAPYVANQGWYDITKT FNGKDDLLCGAATAGNMLHWWFDQNKDQIKRYLKEHPEKQKINFRGEQMFDVKE AIRTKNHQLDSKLFEYFKEKAFPYLSTKHLGVFPDHVIDMFINGYRLSLTNHGPTPVK EGSKDPRGGIFDAVFTRGNQSKLLTSRHDFKEKNLKEISDLIKSELENGKALGLSHTY ANVRINHVINLWGADFDSNGNLKAIYVTDSDSNASIGMKKYFVGVNKAGKVAISAK EIKEDNIGAQVLGLFTLSTGQDSWNQTN |
| 15 | CN107532156A pCART189 |
| | DSFSANQEIRYSEVTPYHVTSVWTKGVTPPANFTQGEDVFHAPYVANQGWYDITKT FNGKDDLLCGAATAGNMLHWWFDQNKDQIKRYLKEHPEKQKINFRGEQMFDVKE AIRTKNHQLDSKLFEYFKEKAFPYLSTKHLGVFPDHVIDMFINGYRLSLTNHGPTPVK KGSKDPRGGIFDAVFTRGNQSKLLTSRHDFKEKNLKEISDLIKKELEEGKALGLSHTY ANVRINHVINLWGADFDSNGNLKAIYVTDSDSNASIGMKKYFVGVNSAGKVAISAK EIKEDNIGAQVLGLFTLSTGQDSWNQTN |
| 16 | CN107532156A pCART190 |
| | DSFSANQEIRYSEVTPYHVTSVWTKGVTPPANFTQGEDVFHAPYVANQGWYDITKT FNGKDDLLCGAATAGNMLHWWFDQNKDQIERYLEEHPEKQKINFRGEQMFDVKE AIDTKNHQLDSKLFEYFKEKAFPYLSTKHLGVFPDHVIDMFINGYRLSLTNHGPTPV KKGSKDPRGGIFDAVFTRGNQSKLLTSRHDFKEKNLKEISDLIKEELTKGKALGLSHT YANVRINHVINLWGADFDSNGNLKAIYVTDSDSNASIGMKKYFVGVNSAGKVAISA KEIKEDNIGAQVLGLFTLSTGQKSWNQTN |
| 17 | CN107532156A pCART209 |
| | DSFSANQEIRYSEVTPYHVTSVWTKGVTPPANFTQGEDVFHAPYVANQGWYDITKT FNGKDDLLCGAATAGNMLHWWFDQNKDQIKRYLEEHPEKQKINFNGEQMFDVKE AIDTKNHQLDSKLFEYFKEKAFPYLSTKHLGVFPDHVIDMFINGYRLSLTNHGPTPV KEGSKDPRGGIFDAVFTRGDQSKLLTSRHDFKEKNLKEISDLIKKELTEGKALGLSHT YANVRINHVINLWGADFDSNGNLKAIYVTDSDSNASIGMKKYFVGVNSAGKVAISA |
| | KEIKEDNIGAQVLGLFTLSTGQDSW |
| 18 | CN107532156A pCART125 |
| | SVWTKGVTPPANFTQGEDVFHAPYVANQGWYDITKTFNGKDDLLCGAATAGNML HWWFDQNKDQIKRYLEEHPEKQKINFNGEQMFDVKEAIDTKNHQLDSKLFEYFKEK AFPYLSTKHLGVFPDHVIDMFINGYRLSLTNHGPTPVKEGSKDPRGGIFDAVFTRGD QSKLLTSRHDFKEKNLKEISDLIKKELTEGKALGLSHTYANVRINHVINLWGADFDS NGNLKAIYVTDSDSNASIGMKKYFVGVNSAGKVAISAKEIKEDNIGAQVLGLFTLST GQDSWNQTN |
| 19 | CN107532156A pCART213 |
| | SVWTKGVTPPANFTQGEDVFHAPYVANQGWYDITKTFNGKDDLLCGAATAGNML HWWFDQNKDQIKRYLEEHPEKQKINFKGEQMFDVKEAIDTKNHQLDSKLFEYFKEK AFPYLSTKHLGVFPDHVIDMFINGYRLSLTNHGPTPVKEGSKDPRGGIFDAVFTRGN QSKLLTSRHDFKEKNLKEISDLIKKELTEGKALGLSHTYANVRINHVINLWGADFDS NGNLKAIYVTDSDSNASIGMKKYFVGVNSAGKVAISAKEIKEDNIGAQVLGLFTLST GQDSWNQTN |
| 20 | CN107532156A pCART214 |
| | SVWTKGVTPPANFTQGEDVFHAPYVANQGWYDITKTFNGKDDLLCGAATAGNML HWWFDQNKDQIERYLEEHPEKQKINFKGEQMFDVKKAIDTKNHQLDSKLFEYFKEK AFPYLSTKHLGVFPDHVIDMFINGYRLSLTNHGPTPVKEGSKDPRGGIFDAVFTRGN QSKLLTSRHDFKEKNLKEISDLIKEELTKGKALGLSHTYANVRINHVINLWGADFDS NGNLKAIYVTDSDSNASIGMKKYFVGVNSAGKVAISAKEIKEDNIGAQVLGLFTLST GQKSWNQTN |
| 21 | CN107532156A pCART228 |
| | DDYQRNATEAYAKEVPHQITSVWTKGVTPPANFTQGEDVFHAPYVANQGWYDITK TFNGKDDLLCGAATAGNMLHWWFDQNKDQIERYLEEHPEKQKINFKGEQMFDVK KAIDTKNHQLDSKLFEYFKEKAFPYLSTKHLGVFPDHVIDMFINGYRLSLTNHGPTP VKEGSKDPRGGIFDAVFTRGNQSKLLTSRHDFKEKNLKEISDLIKEELTKGKALGLSH TYANVRINHVINLWGADFDSNGNLKAIYVTDSDSNASIGMKKYFVGVNSAGKVAIS AKEIKEDNIGAQVLGLFTLSTGQKSWNQTNGGGHHHHHH |
| 22 | CN 107532158 A, Sequence of hybrid IdeS/Z) |
| | DDYQRNATEAYAKEVPHQITSVWTKGVTPLTPEQFRYNNEDVFHAPYVANQGWYD ITKAFDGKDNLLCGAATAGNMLHWWFDQNKDQIKRYLEEHPEKQKINFNGDNMFD VKKAIDTKNHQLDSKLFNYFKEKAFPGLSARRIGVFPDHVIDMFINGYRLSLTNHGP TPVKEGSKDPRGGIFDAVFTRGNQSKLLTSRHDFKNKNLNDISTIIKQELTKGKALGL SHTYANVSINHVINLWGADFNAEGNLEAIYVTDSDSNASIGMKKYFVGVNAHGHVA ISAKKIEGENIGAQVLGLFTLSTGQDSWQKLS |
| 23 | CN107532158A pCART197 |
| | DDYQRNATEAYAKEVPHQITSVWTKGVTPLTPEQFRYNNEDVIHAPYLANQGWYDI TKAFDGKDNLLCGAATAGNMLHWWFDQNKTEIEAYLSKHPEKQKIIFRNQELFDLK |
| | EAIRTKDSQTNSQLFEYFRDKAFPYLSARQLGVMPDLVLDMFINGYYLNVFKTQSTD VKRPYQDKDKRGGIFDAVFTRGNQTTLLTARHDLKNKGLNDISTIIKEELTKGRALA LSHTYANVSISHVINLWGADFNAEGNLEAIYVTDSDANASIGMKKYFVGINKHGHV AISAKKIEGENIGAQVLGLFTLSSGKDIWQKLN |
| 24 | CN107532158A pCART198 |
| | DDYQRNATEAYAKEVPHQITSVWTKGVTPLTPEQFRYNNEDVIHAPYLAHQGWYDI TKTFNGKDNLLCGAATAGNMLHWWFDQNKTEIEAYLSKHPEKQKIIFNNEELFDLK AAIDTKDSQTNSQLFNYFKEKAFPNLSTRQLGVMPDLVLDMFINGYYLNVFKTQST DVNRPYQDKDKRGGIFDAVFTRGNQTTLLTARHDFKEKGLKDISTIIKQELTEGRAL ALSHTYANVSISHVINLWGADFDAEGNLKAIYVTDSDANASIGMKKYFVGINAHGK VAISAKKIEGENIGAQVLGLFTLSSGKDIWQQLS |
| 25 | CN107532158A pCART200 |
| | DSFSANQEIRYSEVTPYHVTSVWTKGVTPLTPEQFRYNNEDVIHAPYLAHQGWYDIT KAFDGKDNLLCGAATAGNMLHWWFDQNKTEIEAYLSKHPEKQKIIFNNQELFDLK AAIDTKDSQTNSQLFNYFRDKAFPNLSARQLGVMPDLVLDMFINGYYLNVFKTQST DVNRPYQDKDKRGGIFDAVFTRGDQTTLLTARHDLKNKGLNDISTIIKQELTEGRAL ALSHTYANVSISHVINLWGADFNAEGNLEAIYVTDSDANASIGMKKYFVGINAHGH VAISAKKIEGENIGAQVLGLFTLSSGKDIWQKLS |
| 26 | CN107532158A pCART201 |
| | SVWTKGVTPLTPEQFRYNNEDVIHAPYLAHQGWYDITKAFDGKDNLLCGAATAGN MLHWWFDQNKTEIEAYLSKHPEKQKIIFNNQELFDLKAAIDTKDSQTNSQLFNYFRD KAFPNLSARQLGVMPDLVLDMFINGYYLNVFKTQSTDVNRPYQDKDKRGGIFDAVF TRGDQTTLLTARHDLKNKGLNDISTIIKQELTEGRALALSHTYANVSISHVINLWGAD FNAEGNLEAIYVTDSDANASIGMKKYFVGINAHGHVAISAKKIEGENIGAQVLGLFT LSSGKDIWQKLS |
| 27 | CN107532158A pCART202 |
| | DDYQRNATEAYAKEVPHQITSVWTKGVTPLTPEQFTQGEDVIHAPYLAHQGWYDIT KAFDGKDNLLCGAATAGNMLHWWFDQNKTEIEAYLSKHPEKQKIIFNNQELFDLK AAIDTKDSQTNSQLFNYFRDKAFPNLSARQLGVMPDLVLDMFINGYYLNVFKTQST DVNRPYQDKDKRGGIFDAVFTRGDQTTLLTARHDLKNKGLNDISTIIKQELTEGRAL ALSHTYANVSISHVINLWGADFNAEGNLEAIYVTDSDANASIGMKKYFVGINAHGH VAISAKKIEGENIGAQVLGLFTLSSGKDIWQKLS |
| 28 | CN107532158A pCART203 |
| | DDYQRNATEAYAKEVPHQITSVWTKGVTPPEQFTQGEDVIHAPYLAHQGWYDITKA FDGKDNLLCGAATAGNMLHWWFDQNKTEIEAYLSKHPEKQKIIFNNQELFDLKAAI DTKDSQTNSQLFNYFRDKAFPNLSARQLGVMPDLVLDMFINGYYLNVFKTQSTDVN RPYQDKDKRGGIFDAVFTRGDQTTLLTARHDLKNKGLNDISTIIKQELTEGRALALS HTYANVSISHVINLWGADFNAEGNLEAIYVTDSDANASIGMKKYFVGINAHGHVAIS AKKIEGENIGAQVLGLFTLSSGKDIWQKLS |
| 29 | CN107532158A pCART204 |
| | DDYQRNATEAYAKEVPHQITSVWTKGVTPPEQFRYNNEDVIHAPYLAHQGWYDIT KAFDGKDNLLCGAATAGNMLHWWFDQNKTEIEAYLSKHPEKQKIIFNNQELFDLK AAIDTKDSQTNSQLFNYFRDKAFPNLSARQLGVMPDLVLDMFINGYYLNVFKTQST DVNRPYQDKDKRGGIFDAVFTRGDQTTLLTARHDLKNKGLNDISTIIKQELTEGRAL ALSHTYANVSISHVINLWGADFNAEGNLEAIYVTDSDANASIGMKKYFVGINAHGH VAISAKKIEGENIGAQVLGLFTLSSGKDIWQKLS |
| 30 | CN107532158A pCART 206 |
| | DDYQRNATEAYAKEVPHQITSVWTKGVTPPEQFTQGEDVIHAPYLAHQGWYDITKA FDGKDNLLCGAATAGNMLHWWFDQNKTEIEAYLSKHPEKQKIIINNQELFDLKAAI DTKDSQTNSQLFNYFRDKAFPNLSARQLGVMPDLVLDMFINGYYLNVFKTQSTDVN RPYQDKDKRGGIFDAVFTRGDQTTLLTARHDLKNKGLNDISTIIKQELTEGRALALS HTYANVSISHVINLWGADFNAEGNLEAIYVTDSDANASIGMKKYFVGINAHGHVAIS AKKIEGENIGAQVLGLFTLSSGKDIWQKLS |
| 31 | CN107532158A pCART207 |
| | DDYQRNATEAYAKEVPHQITSVWTKGVTPPEQFTQGEDVIHAPYLAHQGWYDITKA FDGKDNLLCGAATAGNMLHWWFDQNKTEIEAYLSKHPEKQKIIFRNQELFDLKAAI DTKDSQTNSQLFNYFRDKAFPNLSARQLGVMPDLVLDMFINGYYLNVFKTQSTDVN RPYQDKDKRGGIFDAVFTRGDQTTLLTARHDLKNKGLNDISTIIKQELTEGRALALS HTYANVSISHVINLWGADFNAEGNLEAIYVTDSDANASIGMKKYFVGINAHGHVAIS AKKIEGENIGAQVLGLFTLSSGKDIWQKLS |
| 32 | CN107532158A pCART208 |
| | DDYQRNATEAYAKEVPHQITSVWTKGVTPPEQFTQGEDVIHAPYLAHQGWYDITKA FDGKDNLLCGAATAGNMLHWWFDQNKTEIEAYLSKHPEKQKIIIRNQELFDLKAAI DTKDSQTNSQLFNYFRDKAFPNLSARQLGVMPDLVLDMFINGYYLNVFKTQSTDVN RPYQDKDKRGGIFDAVFTRGDQTTLLTARHDLKNKGLNDISTIIKQELTEGRALALS HTYANVSISHVINLWGADFNAEGNLEAIYVTDSDANASIGMKKYFVGINAHGHVAIS AKKIEGENIGAQVLGLFTLSSGKDIWQKLS |
| 33 | CN107532158A pCART210 |
| | DDYQRNATEAYAKEVPHQITSVWTKGVTPPEQFTQGEDVIHAPYLANQGWYDITKA FDGKDNLLCGAATAGNMLHWWFDQNKTEIEAYLSKHPEKQKIIFRNQELFDLKEAI RTKDSQTNSQLFEYFRDKAFPYLSARQLGVMPDLVLDMFINGYYLNVFKTQSTDVK RPYQDKDKRGGIFDAVFTRGNQTTLLTARHDLKNKGLNDISTIIKEELTKGRALALS HTYANVSISHVINLWGADFNAEGNLEAIYVTDSDANASIGMKKYFVGINKHGHVAIS AKKIEGENIGAQVLGLFTLSSGKDIWQKLN |
| 34 | CN107532158A pCART217 |
| | SVWTKGVTPPEQFTQGEDVIHAPYLAHQGWYDITKAFDGKDNLLCGAATAGNMLH WWFDQNKTEIEAYLSKHPEKQKIIFRNQELFDLKAAIDTKDSQTNSQLFNYFRDKAF PNLSARQLGVMPDLVLDMFINGYYLNVFKTQSTDVNRPYQDKDKRGGIFDAVFTRG |
| | NQTTLLTARHDLKNKGLNDISTIIKQELTEGRALALSHTYANVSISHVINLWGADFNA EGNLEAIYVTDSDANASIGMKKYFVGINAHGHVAISAKKIEGENIGAQVLGLFTLSSG KDIWQKLS |
| 35 | CN107532158A pCART219 |
| | DDYQRNATEAYAKEVPHQITSVWTKGVTPPEQFTQGEDVIHAPYLAHQGWYDITKA FDGADNLLCGAATAGNMLHWWFDQNKTEIEAYLSKHPEKQKIIFRNQELFDLKAAI DTKDSQTNSQLFNYFRDKAFPNLSARQLGVMPDLVLDMFINGYYLNVFKTQSTDVN RPYQDKDKRGGIFDAVFTRGNQTTLLTARHDLKNKGLNDISTIIKQELTEGRALALS HTYANVSISHVINLWGADFNAEGNLEAIYVTDSDANASIGMKKYFVGINAHGHVAIS AKKIEGENIGAQVLGLFTLSSGKDIWQKLS |
| 36 | CN107532158A pCART226 |
| | SVWTKGVTPPEQFTQGEDVIHAPYLAHQGWYDITKAFDGADNLLCGAATAGNMLH WWFDQNKTEIEAYLSKHPEKQKIIFRNQELFDLKAAIDTKDSQTNSQLFNYFRDKAF PNLSARQLGVMPDLVLDMFINGYYLNVFKTQSTDVNRPYQDKDKRGGIFDAVFTRG NQTTLLTARHDLKNKGLNDISTIIKQELTEGRALALSHTYANVSISHVINLWGADFNA EGNLEAIYVTDSDANASIGMKKYFVGINAHGHVAISAKKIEGENIGAQVLGLFTLSSG KDIWQKLS |
| 37 | CN107532158A pCART229 |
| | DDYQRNATEAYAKEVPHQITSVWTKGVTPPEQFTQGEDVIHAPYLAHQGWYDITKA FDGKDNLLCGAATAGNMLHWWFDQNKTEIEAYLSKHPEKQKIIFRNQELFDLKAAI DTKDSQTNSQLFNYFRDKAFPNLSARQLGVMPDLVLDMFINGYYLNVFKTQSTDVN RPYQDKDKRGGIFDAVFTRGNQTTLLTARHDLKNKGLNDISTIIKQELTEGRALALS HTYANVSISHVINLWGADFNAEGNLEAIYVTDSDANASIGMKKYFVGINAHGHVAIS AKKIEGENIGAQVLGLFTLSSGKDIWQKLS |
| 38 | CN107532158A pCARTI91 |
| | DDYQRNATEAYAKEVPHQITSVWTKGVTPLTPEQFTQGEDVFHAPYVANQGWYDI TKAFDGKDNLLCGAATAGNMLHWWFDQNKDQIKRYLEEHPEKQKINFNGENMFD VKKAIDTKNHQLDSKLFNYFKEKAFPYLSAKHLGVFPDHVIDMFINGYRLSLTNHGP TPVKEGSKDPRGGIFDAVFTRGNQSKLLTSRHDFKNKNLNDISTIIKQELTKGKALGL SHTYANVRINHVINLWGADFNAEGNLEAIYVTDSDSNASIGMKKYFVGVNAHGHV AISAKKIEGENIGAQVLGLFTLSTGQDSWQKLS |
| 39 | CN107532158A pCART192 |
| | DDYQRNATEAYAKEVPHQITSVWTKGVTPLTPEQFTQGEDVFHAPYVANQGWYDI TKAFDGKDNLLCGAATAGNMLHWWFDQNKDQIKRYLEEHPEKQKINFRGENMFD VKEAIRTKNHQLDSKLFEYFKEKAFPYLSAKHLGVFPDHVIDMFINGYRLSLTNHGP TPVKKGSKDPRGGIFDAVFTRGNQSKLLTSRHDFKNKNLNDISTIIKSELTNGKALGL SHTYANVRINHVINLWGADFNAEGNLEAIYVTDSDSNASIGMKKYFVGVNKHGHV AISAKKIEGENIGAQVLGLFTLSTGQDSWQKLN |
| 40 | CN107532158A pCARTI93 |
| | DDYQRNATEAYAKEVPHQITSVWTKGVTPLTPEQFTQGEDVFHAPYVANQGWYDI TKTFNGKDDLLCGAATAGNMLHWWFDQNKDQIKRYLEEHPEKQKINFNGEQMFD VKEAIDTKNHQLDSKLFEYFKEKAFPYLSTKHLGVFPDHVIDMFINGYRLSLTNHGP TPVKEGSKDPRGGIFDAVFTRGNQSKLLTSRHDFKEKNLKEISDLIKQELTEGKALGL SHTYANVRINHVINLWGADFDAEGNLKAIYVTDSDSNASIGMKKYFVGVNAAGKV AISAKKIEGENIGAQVLGLFTLSTGQDSWNQTS |
| 41 | CN107532158A pCART194 |
| | DDYQRNATEAYAKEVPHQITSVWTKGVTPLTPEQFTQGEDVFHAPYVANQGWYDI TKTFNGKDDLLCGAATAGNMLHWWFDQNKDQIKRYLEEHPEKQKINFRGEQMFD VKEAIRTKNHQLDSKLFEYFKEKAFPYLSTKHLGVFPDHVIDMFINGYRLSLTNHGP TPVKKGSKDPRGGIFDAVFTRGNQSKLLTSRHDFKEKNLKEISDLIKEELTKGKALGL SHTYANVRINHVINLWGADFDAEGNLKAIYVTDSDSNASIGMKKYFVGVNKAGKV AISAKKIEGENIGAQVLGLFTLSTGQDSWNQTN |
| 42 | CN107532158A pCART205 |
| | DDYQRNATEAYAKEVPHQITSVWTKGVTPPEQFTQGEDVIHAPYVANQGWYDITK AFDGKDNLLCGAATAGNMLHWWFDQNKDQIKRYLEEHPEKQKINFRGEQMFDVK KAIDTKNHQLDSKLFNYFKEKAFPGLSARRIGVFPDHVIDMFINGYRLSLTNHGPTPV KEGSKDPRGGIFDAVFTRGNQSKLLTSRHDFKNKNLNDISTIIKQELTKGKALGLSHT YANVSINHVINLWGADFNAEGNLEAIYVTDSDSNASIGMKKYFVGVNAHGHVAISA KKIEGENIGAQVLGLFTLSTGQDSWQKLS |
| 59 | MMP3 |
| | FRTFPGIPKWRKTHLTYRIVNYTPDLPKDAVDSAVEKALKVWEEVTPLTFSRLYEGE ADIMISFAVREHGDFYPFDGPGNVLAHAYAPGPGINGDAHFDDDEQWTKDTTGTNL FLVAAHEIGHSLGLFHSANTEALMYPLYHSLTDLTRFRLSQDDINGIQSLYGPPPDSP ET |
| 60 | MMP12 |
| | FREMPGGPVWRKHYITYRINNYTPDMNREDVDYAIRKAFQVWSNVTPLKFSKINTG MADILVVFARGAHGDFHAFDGKGGILAHAFGPGSGIGGDAHFDEDEFWTTHSGGTN LFLTAVHEIGHSLGLGHSSDPKAVMFPTYKYVDINTFRLSADDIRGIQSLYGDPKENQ RL |

The mutant described in the present invention is preferably produced by means of genetic engineering recombination.

Preferably, the mutant comprises or consists of any amino acid sequence that has at least 70%, 75%, 80%, 85%, 90%, 95%, or 99% identity to the sequence set forth in SEQ ID NO: 5, and has IgG cysteine protease activity.

A modified immunoglobulin degrading enzyme is obtained by means of modification using a modification method selected from glycosylation, sialylation, albumination, farnesylation, carboxylation, hydroxylation, phosphorylation, and conjugation to a polymer.

As for the modified immunoglobulin degrading enzyme, the modified polypeptide is obtained by modification using conjugation to dextran, PEG, a multimerization domain, a toxin, a detectable label, or a drug.

The modified immunoglobulin degrading enzyme is an HSA fusion protein or an Fc fusion protein.

### 1.2 Fc agent (i.e., an Fc-containing protein or preparation, which will be described below with the Fc-containing protein as an example)

Preferably, in the pharmaceutical combination as described above, the Fc-containing protein is selected from one or more of an antibody, an immunoglobulin, an immunoadhesin, a therapeutic agent, a diagnostic agent, an imaging agent, and an antibody-drug conjugate.

Preferably, the pharmaceutical combination as described above further comprises a leukocyte depleting agent. The leukocyte therein is preferably B cell. The leukocyte depleting agent is preferably an antibody that specifically binds to CD4, CD10, CD19, CD20, CD21, CD22, CD23, CD24, CD37, CD53, CD69, CD70, CD72, CD74, CD75, CD77, CD79a, CD79b, CD80, CD81, CD82, CD83, CD84, CD85, CD86, HLA-DRA, TNFRSF5, TNFRSF6, TNFSF5, TNFSF6, BLR1, HDAC4, HDAC5, HDAC7A, HDAC9, ICOSL, IGBP1, MS4A1, RGS1, SLA2, CD81, IFNB1, IL10, TNFRSF5, TNFRSF7, TNFSF5, AICDA, BLNK, GALNAC4S-6ST, HDAC4, HDAC5, HDAC7A, HDAC9, IL4, INHA, INHBA, KLF6, TNFRSF7, DPP4, FCER2, IL2RA, TNFRSF8, TNFSF7, CR2, IL1R2, ITGA2, ITGA3, MS4A1, ST6GAL1, CD1C, CHST10, HLA-A, HLA-DRA, or NT5E.

Preferably, in the pharmaceutical combination as described above, the Fc-containing protein is an antibody against one selected from CD3, CD11a, CD14, CD25, CD28, CD30, CD33, CD36, CD38, CD40, CD44, CD52, CD55, CD59, CD56, CD103, CD134, CD137, CD138, CD152, CD3, IL-1β, IL-2R, IL-6, IL-12, IL-23, C5, BAFF, BLyS, BCMA, CXCR-4, ICAM-1, SLAMF7/CS1, TNFα, and IgE.

Preferably, in the pharmaceutical combination as described above, the Fc-containing protein is an antibody against one selected from IL1β, IL2, IL2R, IL4, IL6, IL10, IL11, IL12, IL23, C5, BAFF, BLyS, BCMA, CXCR-4, ICAM-1, SLAMF7/CS1, TNFα, IgE, and B7.1/B7.2.

Preferably, in the pharmaceutical combination as described above, the Fc-containing protein is rituximab (CD20), dacizumab (IL2R), basiliximab (IL2R), muromonab-CD3, infliximab (TNFα), adalimumab (TNFα), omalizumab (IgE), efalizumab (CD11a), natalizumab (Integrin α4), tocilizumab (IL6), eculizumab (C5), golimumab (TNFα), canakinumab (IL1β), ustekinumab (IL12/IL23), belimumab (BLyS), cetuximab, bevacizumab, elotuzumab (SLAMF7), or a combination thereof.

The antibody that specifically recognizes CD38 has binding specificity to a human effector cell, and/or the antibody is able to kill a CD38-expressing cell by apoptosis, and/or antibody-dependent cell-mediated cytotoxicity, and/or complement-dependent cytotoxicity.

In the immunoconjugate of the anti-CD38 antibody, the antibody is a monomeric IgM antibody linked to a cytotoxic agent, a radioisotope, or a drug.

The anti-CD38 antibody has binding specificity to CD3, CD4, CD138, IL-15R, membrane- or receptor-bound TNF-α, a human Fc receptor, or membrane- or receptor-bound IL-15.

The anti-CD38 antibody is selected from daratumumab, isatuximab, MOR202, TAK-079, TAK-573, SAR442085, TAK-169, T-007, AMG424, GBR1324, Hexabody-CD38, TSK011010, STI-5171, anti-CD38/IGF-1R bsAb, Anti-CD38 SIFbody, Actinium-225 dara, STI-6129, Anti-CD38/anti-CD3, CD38 TCE, and Y-150.

The anti-CD38 antibody is characterized in that the antibody comprises at least one heavy chain and at least one light chain, wherein the light chain comprises three continuous complementarity determining regions as set forth in the amino acid sequences of SEQ ID NO: 43, SEQ ID NO: 44, and SEQ ID NO: 45, or three continuous complementarity determining regions as set forth in the amino acid sequences of SEQ ID NOs: 49-51; the heavy chain comprises three continuous complementarity determining regions as set forth in the amino acid sequences of SEQ ID NOs: 46-48, or three continuous complementarity determining regions as set forth in the amino acid sequences of SEQ ID NO: 52, SEQ ID NO: 53, and SEQ ID NO: 54; and the epitope-binding fragment is Fab, Fab', F(ab')₂, scFv, or a disulfide-linked Fv fragment.

Preferably, the light chain comprises an amino acid sequence as set forth in SEQ ID NO: 55 or SEQ ID NO: 57, and the heavy chain comprises an amino acid sequence as set forth in SEQ ID NO: 56 or SEQ ID NO: 58;
more preferably, the amino acid sequence of the light chain is as set forth in SEQ ID NO: 55, and the amino acid sequence of the heavy chain is as set forth in SEQ ID NO: 56; or the amino acid sequence of the light chain is as set forth in SEQ ID NO: 57, and the amino acid sequence of the heavy chain is as set forth in SEQ ID NO: 58.

**Table 2. Antibody-related sequences**

| SEQ ID NO: | Sequence |
|---|---|
| 43 | Light chain CDR1: RASQSVSSYLA |
| 44 | Light chain CDR2: DASNRAT |
| 45 | Light chain CDR3: QQRSNWPPTF |
| 46 | Heavy chain CDR1: SFAMS |
| 47 | Heavy chain CDR2: AISGSGGGTYYADSVKG |
| 48 | Heavy chain CDR3: DKILWFGEPVFDY |
| 49 | Light chain CDR1: KASQDVSTWA |
| 50 | Light chain CDR2: SASYRYI |
| 51 | Light chain CDR3: QQHYSPPYT |
| 52 | Heavy chain CDR1: DYWMQ |
| 53 | Heavy chain CDR2: TIYPGDGDTGYAQKFQG |
| 54 | Heavy chain CDR3: GDYYGSNSLDY |
| 55 | Light chain variable region: |
| | |
| 56 | Heavy chain variable region: |
| | EVQLLESGGGLVQPGGSLRLSCAVSGFTFNSFAMSWVRQAPGKGLEWVSAISGSGG GTYYADSVKGRFTISRDNSKNTLYLQMNSLRAEDTAVYFCAKDKILWFGEPVFDY WGQGTLVTVSSAS |
| 57 | Light chain variable region: |
| | DIVMTQSHLSMSTSLGDPVSITCKASQDVSTVVAWYQQKPGQSPRRLIYSASYRYIG VPDRFTGSGAGTDFTFTISSVQAEDLAVYYCQQHYSPPYTFGGGTKLEIKR |
| 58 | Heavy chain variable region: |
| | QVQLVQSGAEVAKPGTSVKLSCKASGYTFTDYWMQWVKQRPGQGLEWIGTIYPGD GDTGYAQKFQGKATLTADKSSKTVYMHLSSLASEDSAVY |

The Fc-containing protein further comprises an antibody that binds to one or more immune checkpoint targets selected from the following. The immune checkpoint is selected from CTLA-4, PD-1, TIM-3, LAG3, Siglec15, 4-1BB, GITR, OX40, CD40L, CD28, TIGIT, VISTA; and the immune checkpoint antibody is selected from anti-CTLA-4 antibody, anti-PD-1 antibody, anti-TIM-3 antibody, anti-LAG3 antibody, anti-Siglec15 antibody, anti-4-1BB antibody, anti-GITR antibody, anti-OX40 antibody, anti-CD40L antibody, anti-CD28 antibody, anti-TIGIT antibody, and anti-VISTA antibody.

### 1.3 Other agents included

The pharmaceutical composition as described above further comprises a chemotherapeutic agent, a hormone agent, and a small molecule targeted preparation. The combined use thereof can cause immunogenic cell death, enhance the antigenicity of tumor cells or the susceptibility to immune cells, and inhibit Treg cells with negative regulation and myeloid-derived suppressor cells (MDSC).

The chemotherapeutic agent is selected from an immunosuppressant, a proteasome inhibitor, a cytotoxic drug, and a cell cycle non-specific drug, wherein the immunosuppressant is, for example, cyclophosphamide, thalidomide, pomalidomide, or lenalidomide; the proteasome inhibitor is, for example, bortezomib; the cytotoxic drug is, for example, gemcitabine, temozolomide, or cisplatin; and the cell cycle non-specific drug is, for example, melphalan, mitoxantrone, vincristine, or doxorubicin.

The hormone agent is, for example, dexamethasone or prednisone.

The small molecule targeted preparation is selected from an epigenetic drug, an inhibitor targeting PI3K/Akt/mTOR signaling pathway, and a tyrosine kinase inhibitor. The tyrosine kinase (PTK) inhibitor has multiple effects of inhibiting tumor angiogenesis and resisting tumor cell growth. For example, gefitinib is an oral epidermal growth factor receptor tyrosine kinase (EGFR-TK) inhibitor. Inhibition of EGFR-TK can hinder tumor growth, metastasis and angiogenesis, and increase tumor cell apoptosis.

Other therapeutic agents are also comprised, including pamidronate, zoledronic acid, chlorophosphonate, risedronate, ibandronate, etidronate, alendronate, tiludronate, arsenic trioxide, filgrastim, pegylated filgrastim, sargramostim, suberoylanilide hydroxamic acid and SCIO-469, all-trans retinoic acid (ATRA), cytarabine, phenylalanine mustard, a vascular endothelial growth factor inhibitor, an immunosuppressant antibody that binds to CD40, a TOR inhibitor, an immunosuppressant antibody that binds to IL-6, and an IGF-IR inhibitor.

Other therapeutic agents are also comprised, including antagonists of epidermal growth factor (EGF), fibroblast growth factor (FGF), hepatocyte growth factor (HGF), tissue factor (TF), protein C, protein S, platelet-derived growth factor (PDGF), Heregulin, macrophage-stimulating protein (MSP), or vascular endothelial growth factor (VEGF), or antagonists of receptors of epidermal growth factor (EGF), fibroblast growth factor (FGF), hepatocyte growth factor (HGF), tissue factor (TF), protein C, protein S, platelet-derived growth factor (PDGF), Heregulin, macrophage-stimulating protein (MSP), or vascular endothelial growth factor (VEGF).

Other therapeutic agents are also comprised, including an analgesic agent, an anti-inflammatory agent, an antimicrobial agent, an antiamoebic agent, a trichomonacidal agent, an anti-Parkinson agent, an anti-dysentery agent, an antispasmodic agent, an antidepressant, an anti-rheumatic agent, an anti-fungal agent, an anti-hypertensive agent, an antipyretic agent, an antiparasitic agent, an antihistamine agent, an α-adrenergic agonist, an α-blocking agent, an anesthetic agent, a bronchodilating agent, a biocide, a bactericide, a bacteriostatic agent, a β-adrenergic blocking agent, a calcium channel blocking agent, a cardiovascular agent, a contraceptive agent, a decongestant, a diuretic agent, a sedative agent, a diagnostic agent, an electrolyte agent, a hypnotic agent, a hormone, a blood glucose elevating agent, a muscle relaxant, a muscle contracting agent, an ophthalmic agent, a parasympathomimetic drug, a psychological stimulant, a tranquilizer, a sympathomimetic drug, a stabilizing agent, a urinary agent, a vaginal agent, a virucide, a vitamin agent, a non-steroidal anti-inflammatory agent, an angiotensin converting enzyme inhibitor, a polypeptide, a protein, a nucleic acid, pharmaceuticals, organic molecules, and a sleep promoter.

### 1.4 Carriers or excipients in the composition

A pharmaceutical carrier may be a liquid, and the pharmaceutical composition may be in the form of a solution. The liquid carrier is used for the preparation of a solution, a suspension, an emulsion, a syrup, an elixir, and a pressurized composition. The active ingredient can be dissolved or suspended in a pharmaceutically acceptable liquid carrier, such as water, an organic solvent, a mixture of the two, or a pharmaceutically acceptable oil or fat.

A pharmaceutical composition for parenteral administration is sterile, substantially isotonic, and pyrogen-free, and is prepared in accordance with the GMP of the FDA or a similar agency. A viral vector drug can be administered as an injectable dosage form of a solution or suspension thereof, wherein the substance is in a physiologically acceptable diluent and a pharmaceutical carrier (which can be a sterile liquid, such as water, oil, saline, glycerol, or ethanol). In addition, auxiliary substances, such as a wetting agent or an emulsifier, a surfactant and a pH buffering substance, may be present in the composition. Other components of the pharmaceutical composition include those of petroleum, animal, plant, or synthetic origin, such as peanut oil, soybean oil, and mineral oil. Generally, glycols such as propylene glycol or polyethylene glycol are preferred liquid carriers, especially for injectable solutions. The Fc-containing protein may be administered in the form of a depot injection or an implant preparation, and these forms can be formulated to allow for sustained release of the active ingredient. Generally, the composition is prepared into an injectable preparation, i.e., a liquid solution or a suspension, or may also be prepared into a solid form suitable for being dissolved or suspended in a liquid carrier before injection.

In the combination product, the immunoglobulin is fixed on a surface, and the pharmaceutically active agent is free in the solution.

### 1.5 Use of product prepared from the composition

The present invention further provides a product containing an agent for reducing the blood IgG level, wherein the agent comprises an immunoglobulin degrading enzyme and an Fc-containing protein, which, as a combination preparation, are simultaneously, separately or sequentially used in the treatment of hyperglobulinemia and/or the prevention of infection. Preferably, the combination preparation is used in a method for treating hyperglobulinemia.

### 1.6 Kit or kit of parts (which protects a third therapeutic ingredient in addition to immunoglobulin degrading enzyme and Fc-containing protein)

The present invention further provides a kit or kit of parts for preventing or treating hyperglobulinemia. The kit comprises: 1) a therapeutically effective amount of an immunoglobulin degrading enzyme; and 2) a therapeutically effective amount of an Fc-containing protein. The kit can further comprise an additional active ingredient other than 1) and 2).

The kit of parts comprises part A and part B, wherein part A comprises a therapeutically effective amount of a medicament for reducing the blood immunoglobulin level, including IgG degrading enzyme, and part B comprises a therapeutically effective amount of a viral vector drug. The kit of parts may further comprise part C. Furthermore, part C can comprise an additional active ingredient other than 1) and 2).

The kit may comprise instructions relating to the administration of a therapeutically effective amount of a medicament for reducing the blood immunoglobulin level and of a therapeutically effective amount of a viral vector drug (e.g., dosage information and administration interval information).

The present invention further provides a composition, such as but not limited to a package and a kit, and a kit or kit of parts, for use in preventing or treating globulinemia. The kit comprises: 1) an immunoglobulin degrading enzyme; 2) a therapeutically effective amount of a pharmaceutically active agent comprising an anti-CD38 antibody; and 3) a tag with instructions for performing the method disclosed herein. In some embodiments, 1) and 2) are in separate containers or the same container.

### 2. Use of the combination in the preparation of a medicament for treating hyperglobulinemia

In a preferred embodiment, the use of a combination comprising a modified immunoglobulin degrading enzyme and an Fc-containing protein of any of the foregoing in the preparation of a medicament for treating hyperglobulinemia is provided.

In a preferred embodiment, the use of a combination comprising a modified immunoglobulin degrading enzyme and an Fc-containing protein of any of the foregoing in the preparation of a medicament for treating multiple myeloma is provided.

In a preferred embodiment, the use of a kit or kit of parts of any of the foregoing in the preparation of a medicament for treating hyperglobulinemia is provided.

### 2.1 Hyperglobulinemia

Hyperglobulinemia (abnormally elevated IgG) is characterized by a higher than normal level of immunoglobulins in a patient. The immunoglobulins include IgA, IgD, IgE, IgM, and IgG, among which excessive γ immunoglobulin IgG is the most common. Excess immunoglobulins are produced by leukocytes selected from B cells, abnormal B cells, IgM-producing cells, IgE-producing cells, and IgA-producing cells; the globulins include γ-globulins; and the hyperglobulinemia includes malignant monoclonal gammaglobulinemia (such as multiple myeloma, heavy chain disease, malignant lymphoma, chronic lymphocytic leukemia, and macroglobulinemia), primary monoclonal gammaglobulinemia, secondary monoclonal gammaglobulinemia (such as non-lymphoreticular tumors, monocytic leukemia, and cryoglobulinemia), benign M proteinemia, monoclonal gammopathy of undetermined significance (MGUS), Waldenstrom macroglobulinemia, AL amyloidosis, solitary plasmacytoma (osteal or ectosteal), POMES syndrome, reactive plasmacytosis, osteolytic lesions of metastatic cancer, plasmablastic lymphoma, monoclonal gammopathy of renal significance (MGRS), etc., wherein MGRS is renal damage caused by monoclonal immunoglobulin or a fragment thereof, and its hematological change is closer to MGUS. Connective tissue diseases are also included, such as systemic lupus erythematosus, rheumatoid arthritis, scleroderma, and Sjogren's syndrome; liver diseases, chronic viral active hepatitis, occult cirrhosis, and lupoid hepatitis; infectious diseases, tuberculosis, leprosy, kala-azar, infectious mononucleosis, sexually transmitted diseases, lymphogranuloma, actinomycosis, malaria, and trypanosomiasis; and others, such as sarcoidosis, myasthenia gravis, Hodgkin's disease, monocytic leukemia, Behcet's disease, nephritis, allergic purpura, and immune (or spontaneous) thrombocytopenia.

### 2.2 CD38-related cancers or autoimmune diseases

CD38 is a therapeutic target for a variety of hyperglobulinemic hematologic tumors such as multiple myeloma. CD38-related cancers or autoimmune diseases also include leukemia and lymphoma, such as B-cell chronic lymphoblastic leukemia, T-cell and B-cell acute lymphoblastic leukemia, Waldenstrom macroglobulinemia, primary systemic amyloidosis, mantle cell lymphoma, prolymphocytic/myeloid leukemia, acute myeloid leukemia, chronic myeloid leukemia, follicular lymphoma, Burkitt's lymphoma, large granular lymphocyte (LGL) leukemia, NK cell leukemia and plasma cell leukemia, diffuse large B-cell lymphoma (DLBCL), follicular lymphoma (FL), mantle cell lymphoma (MCL), chronic lymphocytic leukemia (CLL), and acute myeloid leukemia (AML). Multiple myeloma is a hematologic tumor caused by plasma cell canceration in bone marrow. At present, there are many Fc agents targeting CD38, including but not limited to daratumumab, isatuximab, MOR202, etc.

The use of the pharmaceutical combination in the preparation of a medicament used in a method for inhibiting the growth and/or proliferation of CD38-expressing cells is provided, the method comprising administering the pharmaceutical composition to inhibit the growth and/or proliferation of the cells.

The use of the pharmaceutical combination in the preparation of a medicament used in a method for treating a disease or symptom caused by the participation of CD38-expressing cells in a patient is provided, the method comprising administering the pharmaceutical composition to the patient in need of such a treatment.

The use of the pharmaceutical combination in the preparation of a medicament used in a method for preventing a disease or symptom caused by the participation of CD38-expressing cells in a patient is provided, the method comprising administering the pharmaceutical composition to the patient in need of such a treatment.

### 2.3 Systemic lupus erythematosus

An immunopathogenic sign of systemic lupus erythematosus (SLE) is activation of polyclonal B cells, which leads to hyperglobulinemia, the production of autoantibodies, and the formation of immune complexes. The main abnormality seems to be due to the fact that generalized T cell disorder leads to the inability of T cells to inhibit these forbidden B cell cloning. In addition, several cytokines (such as IL-10) and costimulatory molecules (such as CD40 and CD40L, B7 and CD28 and CTLA-4) that trigger a second signal also promote the interaction between B cells and T cells. These interactions, along with weakened phagocytic clearance of immune complexes and apoptotic substances, maintain the immune responses and thus cause tissue damage. The combination of the present invention will enhance the therapeutic efficacy against SLE (see Sfikakis PP et. al., 2005 Curr Opin Rheumatol 17:550-7; Peng SL (2004) Methods Mol Med.; 102: 227-72).

### 3. Combination therapy for hyperglobulinemia

In a preferred embodiment, the immunoglobulin degrading enzyme and the Fc agent are used for preventing and/or treating hyperglobulinemia.

The hyperglobulinemia is selected from the group consisting of the following conditions or the following conditions: malignant monoclonal gammaglobulinemia (such as multiple myeloma, heavy chain disease, malignant lymphoma, chronic lymphocytic leukemia, and macroglobulinemia), secondary monoclonal gammaglobulinemia (such as non-lymphoreticular tumors, monocytic leukemia, and cryoglobulinemia), benign M proteinemia, monoclonal gammopathy of undetermined significance (MGUS), Waldenstrom macroglobulinemia, AL amyloidosis, solitary plasmacytoma (osteal or ectosteal), POMES syndrome, reactive plasmacytosis, osteolytic lesions of metastatic cancer, plasmablastic lymphoma, monoclonal gammopathy of renal significance (MGRS), etc. The condition is preferably multiple myeloma.

When the immunoglobulin degrading enzyme is administered before the administration of the Fc agent, the level of immunoglobulins, especially M protein, in the blood of the subject is quantitatively detected before and after the administration of the immunoglobulin degrading enzyme and before and after the administration of the Fc agent.

When the immunoglobulin degrading enzyme is administered after the administration of the Fc agent, the level of immunoglobulins, especially M protein, in the blood of the subject is quantitatively detected before and after the administration of the Fc agent and before and after the administration of the immunoglobulin degrading enzyme.

Preferably, the immunoglobulin degrading enzyme is administered before the administration of the Fc agent, or the immunoglobulin degrading enzyme is administered after the administration of the Fc agent.

In the use of the present invention, the immunoglobulin degrading enzyme is present as a combination preparation for simultaneous, separate or sequential use with the Fc agent.

In some embodiments, the method comprises the following steps: 1) administering the immunoglobulin degrading enzyme to the subject; and then 2) administering the Fc agent to the subject. Preferably, there is a time interval between the administration of the immunoglobulin degrading enzyme and the administration of the Fc agent.

In some embodiments, the method comprises the following steps: 1) administering the Fc agent to the subject; and then 2) administering the immunoglobulin degrading enzyme to the subject. Preferably, there is a time interval between the administration of the immunoglobulin degrading enzyme and the administration of the Fc agent.

Preferably, the administration amount of the immunoglobulin degrading enzyme and the time interval are sufficient to reduce the immunoglobulin level in the subject to 60% of the initial level. More preferably, the administration amount of the agent and the time interval are sufficient to reduce the immunoglobulin level in the subject to less than 50%, 40%, 30%, 20% or 10% of the initial level in the patient. The agent can be administered at a single time point or within a set period of time.

The agent and oncolytic virus or viral vaccine can be administered by any suitable route. Preferably, the immunoglobulin degrading enzyme is administered by intravenous infusion or subcutaneous injection, and/or the amount of the agent administered is 0.01 mg/kg body weight to 2 mg/kg body weight, 0.04 to 2 mg/kg body weight, 0.12 mg/kg body weight to 2 mg/kg body weight, 0.24 mg/kg body weight to 2 mg/kg body weight, or 1 mg/kg body weight to 2 mg/kg body weight.

Preferably, the time interval between the administration of the immunoglobulin degrading enzyme and the administration of the Fc agent is at least 30 min, at least 1 h, at least 2 h, at least 3 h, at least 4 h, at least 4 h, at least 5 h, or at least 6 h; and at most 35 days, at most 28 days, at most 21 days, at most 18 days, at most 14 days, at most 13 days, at most 12 days, at most 11 days, at most 10 days, at most 9 days, at most 8 days, at most 7 days, at most 6 days, at most 5 days, at most 4 days, at most 3 days, at most 2 days, at most 24 h, at most 18 h, at most 12 h, at most 10 h, at most 8 h, at most 7 h, or at most 6 h.

Preferably, the time interval between the administration of the immunoglobulin degrading enzyme and the administration of the Fc agent is 30 min to 1 h, 30 min to 2 h, 30 min to 3 h, 30 min to 4 h, 30 min to 5 h, 30 min to 6 h, 1 to 2 h, 1 to 3 h, 1 to 4 h, 1 to 5 h, 1 to 6 h, 2 to 3 h, 2 to 4 h, 2 to 5 h, 2 to 6 h, 3 to 4 h, 3 to 5 h, 3 to 6 h, 4 to 5 h, 4 to 6 h, or 5 to 6 h.

In yet another embodiment, the method comprises the following steps: 1) treating blood from the subject *in vitro* with the immunoglobulin degrading enzyme; 2) returning the blood to the subject; and 3) administering the Fc agent to the subject.

In yet another embodiment, the method comprises the following steps: 1) administering the Fc agent to the subject; 2) treating blood from the subject *in vitro* with the immunoglobulin degrading enzyme; and 3) returning the blood to the subject.

In a preferred embodiment, the immunoglobulin degrading enzyme and the Fc agent are used for treating hyperglobulinemia.

In a preferred embodiment, the immunoglobulin degrading enzyme and the Fc agent are used for preventing hyperglobulinemia.

In any aspect of the present invention, the immunoglobulin degrading enzyme and the Fc agent can be administered simultaneously, separately or sequentially, for use in the treatment and prevention of hyperglobulinemia. The immunoglobulin degrading enzyme and the Fc agent can be provided as separate preparations or as a combination preparation.

On the basis of complying with common knowledge in the art, the above preferred conditions can be arbitrarily combined to obtain various preferred examples of the present invention.

The reagents and raw materials used in the present invention are all commercially available.

The positive progressive effects of the present invention lie in reducing the influence of globulins on treatment by applying an immunoglobulin degrading enzyme in combination with an Fc agent to hyperglobulinemia, so that the Fc agent can function better; moreover, the clinical dosage of some Fc agents can also be reduced, which can reduce the side effects of the therapeutic agents on the one hand and also the economic burden of medication on the other hand.

### Brief Description of the Drawings

Figure 1 shows the SDS-PAGE gel electrophoresis pattern of cleavage products resulting from cleavage of human IgG1 by seven single-point mutants and wild-type IdeE (enzyme : substrate = 1 : 1000).
Figure 2 shows the SDS-PAGE gel electrophoresis pattern of cleavage products resulting from cleavage of human IgG1 by seven single-point mutants and wild-type IdeE (enzyme : substrate = 1 : 2000).
Figure 3 shows the SDS-PAGE gel electrophoresis pattern of cleavage products resulting from cleavage of human IgG1 by five N-terminal truncated mutants (enzyme : substrate = 1 : 1000).
Figure 4 shows the SDS-PAGE gel electrophoresis pattern of cleavage products resulting from cleavage of human IgG1 by five N-terminal truncated mutants and wild-type IdeE after being placed at 50°C for 1 h (enzyme : substrate = 1 : 1000).
Figure 5 shows the SDS-PAGE gel electrophoresis pattern of cleavage products resulting from cleavage of human IgG1 by two C-terminal truncated mutants (enzyme : substrate = 1 : 1000).
Figure 6 shows the SDS-PAGE gel electrophoresis pattern of cleavage products resulting from cleavage of human IgG1 by five combinatorial mutants (enzyme : substrate = 1 : 2000).
Figure 7 shows the SDS-PAGE gel electrophoresis pattern of cleavage products resulting from cleavage of human IgG1 by five combinatorial mutants after being placed at 50°C for 1 h (enzyme : substrate = 1 : 2000).
Figure 8 shows the SDS-PAGE gel electrophoresis pattern of cleavage products resulting from cleavage of human IgG1 by mutant E97D_del18 and IdeS at different concentrations.
Figure 9 shows the SDS-PAGE gel electrophoresis pattern of cleavage products resulting from cleavage of human IgG1 by mutant E97D_del18 and IdeZ at different concentrations.
Figures 10A and 10B show the SDS-PAGE gel electrophoresis patterns of cleavage products resulting from cleavage of human IgG1 by mutants with different mutation combinations and IdeE (enzyme : substrate = 1 : 2000).
Figure 11 shows the SDS-PAGE gel electrophoresis pattern of cleavage products resulting from cleavage of human IVIg by mutant E97D_del18 in the sera and plasmas of mice.
Figure 12 shows the SDS-PAGE gel electrophoresis pattern of cleavage products produced by mutant E97D_del18 in the sera of mice and humans.
Figure 13 shows the SDS-PAGE gel electrophoresis pattern of cleavage products resulting from cleavage of human IVIg by E97D_del18 at different times in mice.
Figure 14 shows that in the tumor model of Daudi cells, the immunoglobulin degrading enzyme reduces IgG's inhibition of tumor suppression by the antibody.

### Examples

The present invention will be further described by way of examples below, but the present invention is not limited to the scope of the described examples. The experimental methods for which no specific conditions are indicated in the following examples are selected according to conventional methods and conditions or according to the product's instructions.

### Example 1. Preparation of protein

### 1) Preparation of anti-CD38 monoclonal antibody liquid preparation

Daratumumab was harvested from a cell culture fluid and purified by immobilized protein A affinity chromatography, cation exchange chromatography (such as SP Sepharose FF), a filtration step to remove viral contaminates, followed by hydrophobic chromatography (such as Butyl Sepharose HP), and an ultrafiltration and buffer exchange step. In order to prepare the pharmaceutical composition according to the example, daratumumab was provided at a concentration of about 100 mg/mL in 20 mM histidine buffer (pH ~6.0). Isatuximab was prepared by the same method.

### 2) Preparation of IdeS and IdeZ

After entrusting GenScript Biotech Corporation to perform codon preference optimization for *E. coli,* polynucleotide sequences encoding SEQ ID NO: 1 to SEQ ID NO: 5 were synthesized, and N-terminal methionine and C-terminal 6×his tags were added. After being synthesized, the sequences were inserted into expression vector pET32a. After being verified to be correct by sequencing, a recombinant plasmid for polypeptide expression was obtained. The prepared recombinant plasmid was electrotransformed into *E. coli* BL21 Star (DE3), and the cells were plated on an LB agarose plate containing 100 µg/mL of ampicillin. The plate was cultured at 37°C overnight until colonies grew out. Single colonies were picked and inoculated to 3 ml of LB medium containing 100 µg/mL of ampicillin, and cultured at 37°C and 250 rpm overnight. The overnight cultures were inoculated to 50 mL of LB medium containing 100 µg/mL of ampicillin, and cultured at 37°C until OD600 reached 0.4-0.6. Then, 0.1 mM of IPTG was added, and the mixtures were further cultured at 20°C overnight. Cell pellets were collected from the overnight cultures by centrifugation, resuspended with PBS to 100 g/L, and then disrupted by ultrasonication. After the cells were disrupted, the supernatant was collected by centrifugation and then purified by IDA-Ni agarose magnetic beads, and the eluted proteins were then buffer exchanged into a PBS buffer system with an ultrafiltration centrifuge tube. SDS-PAGE was used to evaluate the purity of each protein.

### Example 2. Comparison of human IgG1-cleaving activity of single-point mutants

IdeEs T8D (SEQ ID NO: 63), T8W (SEQ ID NO: 65), T24A (SEQ ID NO: 69), A59L (SEQ ID NO: 73), A59V (SEQ ID NO: 74), E97D (SEQ ID NO: 75), and R280H (SEQ ID NO: 76) were synthesized according to the method of Example 1, and these seven single-point mutants were evaluated for their human IgG1-cleaving activity.

The activity of cleaving human IgG1 of different mutants relative to the wild-type IdeE was further evaluated via the cleavage products resulting from cleavage of human IgG1 by each mutant at different concentrations displayed on SDS-PAGE. The purified mutants and the wild-type IdeE were diluted to 0.002 mg/mL and 0.001 mg/mL, respectively. 50 µl of mutants or wild-type IdeE at different concentrations was each added to 50 µl of reaction system containing 2 mg/ml of trastuzumab to initiate the cleavage reaction, and the reaction systems were placed at 37°C for 30 min. The samples were mixed with an equal volume of 2× SDS loading buffer, and the mixtures were then placed in water bath at 75°C for 5 min. The cleavage products were detected by SDS-PAGE.

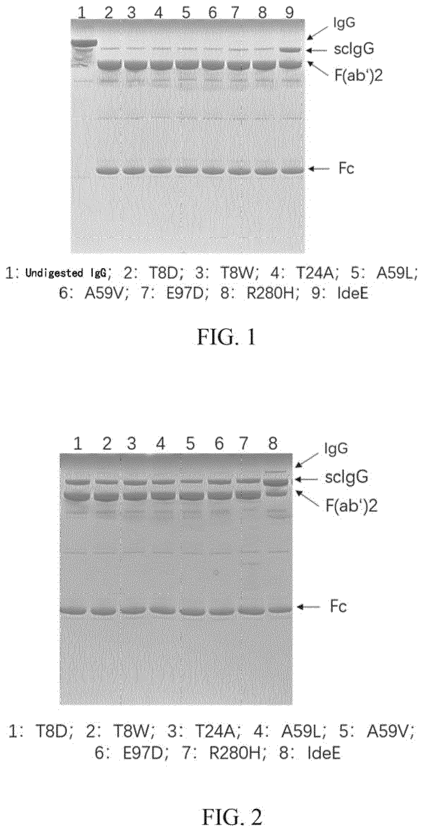

Figure 1 shows the electrophoretogram of cleavage products resulting from cleavage of human IgG1 by the seven single-point mutants and the wild-type IdeE (enzyme : substrate = 1 : 1000). Figure 2 shows the electrophoretogram of cleavage products resulting from cleavage of human IgG1 by the seven single-point mutants and the wild-type IdeE (enzyme : substrate = 1 : 2000). The seven single-point mutants at the concentration of 0.001 mg/ml all cleaved IgG1 with an efficiency not worse than that of 0.002 mg/ml of wild-type IdeE, indicating that the activity of cleaving human IgG1 of the seven single-point mutants is not less than twice that of the wild-type IdeE.

### Example 3. Comparison of human IgG1-cleaving activity and thermal stability of N-terminal truncated mutants

On the basis of the wild-type IdeE, the first 15 amino acids (D1-V15), the first 16 amino acids (D1-P16), the first 17 amino acids (D1-H17), the first 18 amino acids (D1-Q18) and the first 19 amino acids (D1-I19) at its N-terminus were respectively deleted, so as to construct five N-terminal truncated mutants (see Table 3).

**Table 3. Design of sequences of truncated mutants**

| Mutant | Modification relative to wild-type or mutant sequence | SEQ ID NO: |
|---|---|---|
| WT_del15 | Deletion of the first 15 amino acids in SEQ ID NO: 62 | 78 |
| WT_del16 | Deletion of the first 16 amino acids in SEQ ID NO: 62 | 79 |
| WT_del17 | Deletion of the first 17 amino acids in SEQ ID NO: 62 | 80 |
| WT_del18 | Deletion of the first 18 amino acids in SEQ ID NO: 62 | 81 |
| WT_del19 | Deletion of the first 19 amino acids in SEQ ID NO: 62 | 82 |

### 1. Expression and purification of mutants

According to the method of Example 1, the polynucleotide sequences of the mutants in Table 3 were synthesized, mutant expression recombinant plasmids were constructed, *E. coli* BL21 Star (DE3) was transformed therewith, and purified proteins of the mutants were prepared.

### 2. Comparison of human IgG1-cleaving activity of mutants

The purified mutants and the wild-type IdeE were diluted to 0.002 mg/mL, respectively. 50 µl of diluted mutants or wild-type IdeE was each added to 50 µl of reaction system containing 2 mg/ml of trastuzumab to initiate the cleavage reaction, and the reaction systems were placed at 37°C for 30 min. The samples were mixed with an equal volume of 2× SDS loading buffer, and the mixtures were then placed in water bath at 75°C for 5 min. The cleavage products were detected by SDS-PAGE.

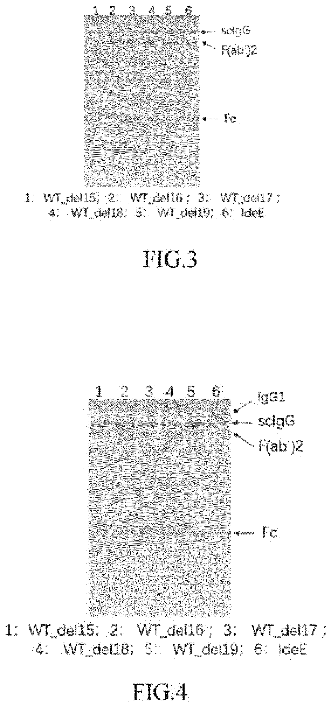

Figure 3 shows the electrophoretogram of cleavage products resulting from cleavage of human IgG1 by the five truncated mutants (enzyme : substrate = 1 : 1000). The cleavage activity of all of the five truncated mutants was not significantly different from that of the wild-type IdeE.

### 3. Comparison of thermal stability of mutants

The purified mutants and the wild-type IdeE were respectively diluted to 0.1 mg/ml, placed at 50°C for 1 h, and then further diluted to 0.002 mg/mL. 50 µl of diluted mutants or wild-type IdeE was each added to 50 µl of reaction system containing 2 mg/ml of trastuzumab to initiate the cleavage reaction, and the reaction systems were placed at 37°C for 30 min. The samples were mixed with an equal volume of 2× SDS loading buffer, and the mixtures were then placed in water bath at 75°C for 5 min. The cleavage products were detected by SDS-PAGE.

Figure 4 shows the electrophoretogram of cleavage products resulting from cleavage of human IgG1 by the five truncated mutants and the wild-type IdeE after being placed at 50°C for 1 h (enzyme : substrate = 1 : 1000). The residual activity of the five truncated mutants after heat treatment at 50°C was significantly higher than that of the wild-type, indicating that the thermal stability of all of the five truncated mutants is significantly improved compared with that of the wild-type.

### Example 4. Comparison of human IgG1-cleaving activity of C-terminal truncated mutants

On the basis of the wild-type IdeE, the last 5 amino acids (W311-S315) and the last 10 amino acids (S306-S315) at its C-terminus were respectively deleted, so as to construct two C-terminal truncated mutants (see Table 4).

**Table 4. Design of sequences of truncated mutants**

| Mutant | Modification relative to wild-type or mutant sequence | SEQ ID NO: |
|---|---|---|
| WT_delC5 | Deletion of the last 5 amino acids in SEQ ID NO: 62 | 83 |
| WT_delC10 | Deletion of the last 10 amino acids in SEQ ID NO: 62 | 84 |

### 1. Expression and purification of mutants

According to the method of Example 1, the polynucleotide sequences of the mutants in Table 4 were synthesized, mutant expression recombinant plasmids were constructed, *E. coli* BL21 Star (DE3) was transformed therewith, and purified proteins of the mutants were prepared.

### 2. Comparison of human IgG1-cleaving activity of mutants

The purified mutants and the wild-type IdeE were diluted to 0.002 mg/mL, respectively. 50 µl of diluted mutants or wild-type IdeE was each added to 50 µl of reaction system containing 2 mg/ml of trastuzumab to initiate the cleavage reaction, and the reaction systems were placed at 37°C for 30 min. The samples were mixed with an equal volume of 2× SDS loading buffer, and the mixtures were then placed in water bath at 75°C for 5 min. The cleavage products were detected by SDS-PAGE.

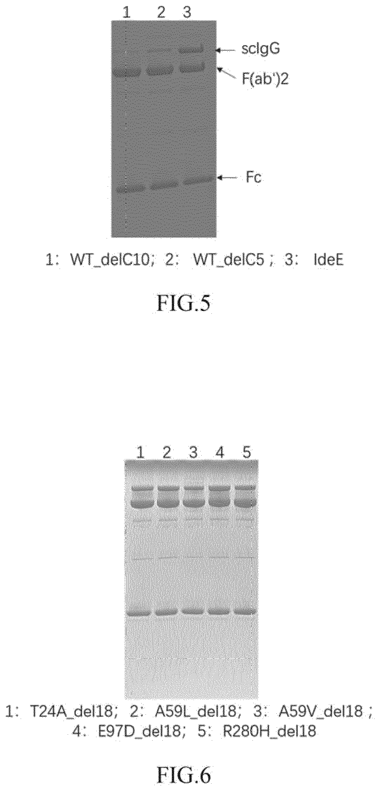

Figure 5 shows the electrophoretogram of cleavage products resulting from cleavage of human IgG1 by the two C-terminal truncated mutants (enzyme : substrate = 1 : 1000). The cleavage activity of both of the two truncated mutants was twice or higher than that of the wild-type IdeE.

### Example 5. Comparison of human IgG1-cleaving activity and thermal stability of combinatorial mutants

On the basis of the five single-point mutants, T24A, A59L, A59V, E97D, and R280H, the first 18 amino acids (D1-Q18) were respectively deleted, so as to construct five combinatorial mutants (see Table 5).

**Table 5. Design of sequences of combinatorial mutants**

| Mutant | Modification relative to wild-type or mutant sequence | SEQ ID NO: |
|---|---|---|
| T24A_del18 | Deletion of the first 18 amino acids in SEQ ID NO: 69 | 85 |
| A59L_del18 | Deletion of the first 18 amino acids in SEQ ID NO: 73 | 86 |
| A59V_del18 | Deletion of the first 18 amino acids in SEQ ID NO: 74 | 87 |
| E97D_del18 | Deletion of the first 18 amino acids in SEQ ID NO: 75 | 88 |
| R280H_del18 | Deletion of the first 18 amino acids in SEQ ID NO: 76 | 89 |

### 1. Expression and purification of mutants

According to the method of Example 1, the polynucleotide sequences of the mutants in Table 5 were synthesized, mutant expression recombinant plasmids were constructed, *E. coli* BL21 Star (DE3) was transformed therewith, and purified proteins of the mutants were prepared.

### 2. Comparison of human IgG1-cleaving activity of mutants

The purified mutants were diluted to 0.001 mg/mL, respectively. 50 µl of diluted mutants or wild-type IdeE was each added to 50 µl of reaction system containing 2 mg/ml of trastuzumab to initiate the cleavage reaction, and the reaction systems were placed at 37°C for 30 min. The samples were mixed with an equal volume of 2× SDS loading buffer, and the mixtures were then placed in water bath at 75°C for 5 min. The cleavage products were detected by SDS-PAGE.

Figure 6 shows the electrophoretogram of cleavage products resulting from cleavage of human IgG1 by the five combinatorial mutants (enzyme : substrate = 1 : 2000). Comparing the cleavage in Figure 6 to that in Figure 2, there was no significant difference in cleavage activity between the five truncated mutants and the five single-point combinatorial mutants, indicating that the activity of cleaving human IgG1 of the combinatorial mutants is also not less than twice that of the wild-type IdeE.

### 3. Comparison of thermal stability of mutants

The purified mutants were respectively diluted to 0.1 mg/ml, placed at 50°C for 1 h, and then further diluted to 0.001 mg/mL. 50 µl of diluted mutants or wild-type IdeE was each added to 50 µl of reaction system containing 2 mg/ml of trastuzumab to initiate the cleavage reaction, and the reaction systems were placed at 37°C for 30 min. The samples were mixed with an equal volume of 2× SDS loading buffer, and the mixtures were then placed in water bath at 75°C for 5 min. The cleavage products were detected by SDS-PAGE.

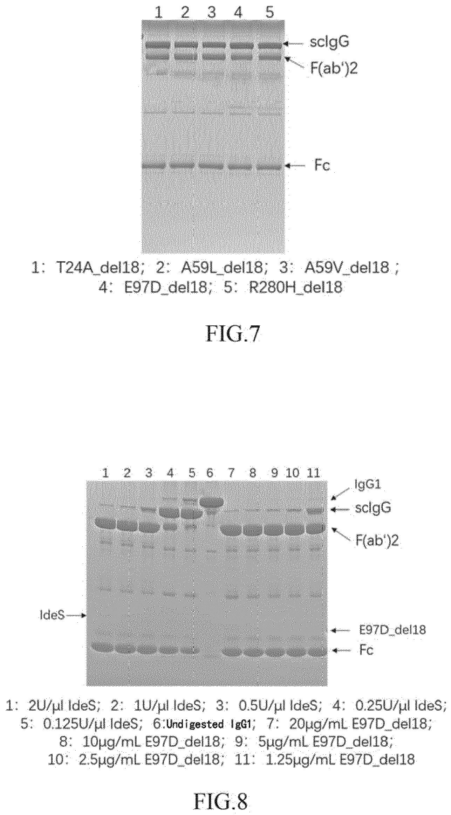

Figure 7 shows the electrophoretogram of cleavage products resulting from cleavage of human IgG1 by the five combinatorial mutants after being placed at 50°C for 1 h (enzyme : substrate = 1 : 2000). Comparing the cleavage in Figure 7 to that in Figure 2, the activity of the five combinatorial mutants after heat treatment at 50°C only decreased slightly, indicating that the thermal stability of all of the five combinatorial mutants is also significantly improved compared with that of the wild-type.

### Example 6. Comparison of activity of mutant E97D del18 to that of IdeS and IdeZ

The purified E97D_del18 mutant in Example 5 was sequentially diluted to 20 µg/mL, 10 µg/mL, 5 µg/mL, 2.5 µg/mL, and 1.25 µg/ml. IdeS (FabRICATOR^{®}, Art.No. A0-FRI-020, Genovis) was respectively diluted to 2 U/µl, 1 U/µl, 0.5 U/µl, 0.25 U/µl, and 0.125 U/µl according to its label. IdeZ (FabRICATOR-Z^{®}, Art.No. A0-FRZ-020, Genovis) was diluted to 0.4 U/µl, 0.2 U/µl, 0.1 U/µl, 0.05 U/µl, and 0.025 U/µl, respectively. 50 µl of mutants, IdeS or IdeZ at different concentrations was each added to 50 µl of reaction system containing 2 mg/ml of trastuzumab to initiate the cleavage reaction, and the reaction systems were placed at 37°C for 30 min. The samples were mixed with an equal volume of 2× SDS loading buffer, and the mixtures were then placed in water bath at 75°C for 5 min. The cleavage products were detected by SDS-PAGE.

Figure 8 shows the electrophoretogram of cleavage products resulting from cleavage of human IgG1 by mutant E97D_del18 and IdeS at different concentrations. From the enzyme protein bands on the electrophoretogram, it can be judged that the concentration of enzyme IdeS in lane 1 was between those of mutant enzyme E97D_del18 in lanes 7 and 8, thus the concentration of enzyme IdeS in lane 3 was deduced to be between those of mutant enzyme E97D_del18 in lanes 9 and 10, and the enzymatic digestion of IgG1 in lane 3 was between those in lanes 10 and 11, and therefore it can be inferred that the activity of cleaving human IgG1 of mutant E97D_del18 is nearly twice that of IdeS.

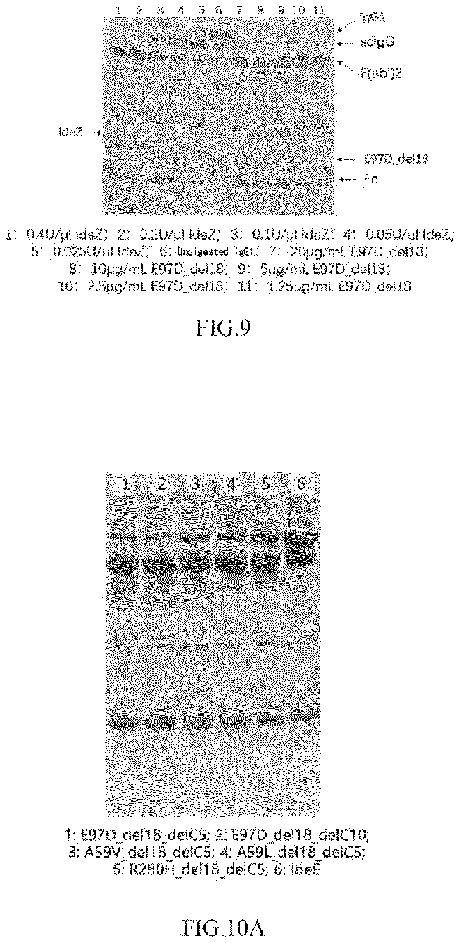

Figure 9 shows the electrophoretogram of cleavage products resulting from cleavage of human IgG1 by mutant E97D_del18 and IdeZ at different concentrations. From the enzyme protein bands on the electrophoretogram, it can be judged that the concentration of enzyme IdeZ in lane 1 was higher than that of mutant enzyme E97D_del18 in lane 7, thus the concentration of enzyme IdeZ in lane 3 was deduced to be higher than that of mutant enzyme E97D_del18 in lane 9, that is, 4 times higher than that of mutant enzyme E97D_del18 in lane 11, and the enzymatic digestion of IgG1 in lane 3 was close to that in lane 11, and therefore it can be inferred that the activity of cleaving human IgG1 of mutant E97D_del18 is 4 times higher than that of IdeZ.

### Example 7. Comparison of human IgG1-cleaving activity of combinatorial mutants

On the basis of mutant E97D_del18, the last 5 amino acids (W311-S315) and the last 10 amino acids (S306-S315) at its C-terminus were deleted, so as to construct two C-terminal truncated mutants and one triple-mutation combination (see Table 6).

**Table 6. Design of sequences of combinatorial mutants**

| Mutant | Modification relative to wild-type or mutant sequence | SEQ ID NO: |
|---|---|---|
| E97D_del18_delC5 | Deletion of the last 5 amino acids in SEQ ID NO: 88 | 90 |
| E97D_del18_delC10 | Deletion of the last 10 amino acids in SEQ ID NO: 88 | 91 |
| A59V_del18_delC5 | Deletion of the last 5 amino acids in SEQ ID NO: 87 | 92 |
| A59L_del18_delC5 | Deletion of the last 5 amino acids in SEQ ID NO: 86 | 93 |
| R280H_del18_delC5 | Deletion of the last 5 amino acids in SEQ ID NO: 89 | 94 |
| E97D_A59V_R280H | Combination of three mutations, E97D, A59V and R280H | 95 |

### 1. Expression and purification of mutants

According to the method of Example 1, the polynucleotide sequences of the mutants in Table 6 were synthesized, mutant expression recombinant plasmids were constructed, *E. coli* BL21 Star (DE3) was transformed therewith, and purified proteins of the mutants were prepared.

### 2. Comparison of human IgG1-cleaving activity of mutants

The purified mutants were diluted to 0.001 mg/mL, respectively. 50 µl of diluted mutants or wild-type IdeE was each added to 50 µl of reaction system containing 2 mg/ml of trastuzumab to initiate the cleavage reaction, and the reaction systems were placed at 37°C for 30 min. The samples were mixed with an equal volume of 2× SDS loading buffer, and the mixtures were then placed in water bath at 75°C for 5 min. The cleavage products were detected by SDS-PAGE.

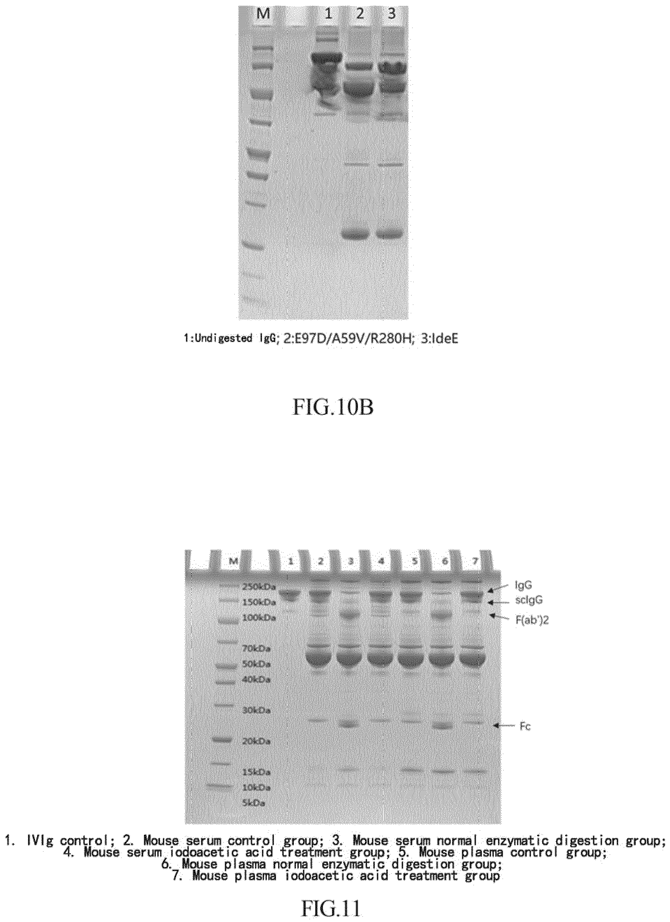

Figures 10A and 10B show the electrophoretograms of cleavage products resulting from cleavage of human IgG1 by the six combinatorial mutants (enzyme : substrate = 1 : 2000).

### Example 7. In vitro detection of human IgG1-cleaving activity of mutant E97D del18

The activity of cleaving human IgG1 *in vitro* of mutant E97D_del18 was evaluated by detecting the amount of intact or single-cleaved IVIg in the sera or plasmas of mice treated with mutant E97D_del18 and human IVIg. According to Table 7, mouse serum or plasma enzymatic digestion systems were formulated for different groups.

**Table 7. Mouse serum or plasma enzymatic digestion systems**

| Group name | Volume of PBS (µl) | Volume of serum or plasma (µl) | Concentration of IVIg in the system (mg/ml) | Concentration of E97D_del18 in the system (mg/ml) | Concentration of iodoacetic acid in the system (mM) |
|---|---|---|---|---|---|
| IVIg control | 100 | / | 10 | / | / |
| Mouse serum control group | / | 100 | 10 | / | / |
| Mouse serum normal enzymatic digestion group | / | 100 | 10 | 0.05 | / |
| Mouse serum iodoacetic acid treatment group | / | 100 | 10 | 0.05 | 2 |
| Mouse plasma control group | / | 100 | 10 | / | / |
| Mouse plasma normal enzymatic digestion group | / | 100 | 10 | 0.05 | / |
| Mouse plasma iodoacetic acid treatment group | / | 100 | 10 | 0.05 | 2 |

The function of iodoacetic acid in the iodoacetic acid treatment groups was to inhibit the activity of the IgG degrading enzyme.

The systems were placed at 37°C for 30 min. 20 µl of samples were mixed with an equal volume of 2× SDS non-reducing loading buffer, and then placed in water bath at 75°C for 5 min. The cleavage products were detected by SDS-PAGE.

Figure 11 shows the electrophoretogram of cleavage products resulting from cleavage of human IVIg by mutant E97D_del18 in the sera and plasmas of mice. The results showed that E97D_del18 can effectively cleave human IVIg in both the sera and plasmas of mice.

Whether mutant E97D_del18 has the activity of cleaving human IgG1 *in vitro* was evaluated by detecting the sera of mice or humans treated with mutant E97D_del18. According to Table 8, mouse or human serum enzymatic digestion systems were formulated for different groups.

**Table 8. Mouse or human serum enzymatic digestion systems**

| Group name | Volume of mouse serum (µl) | Volume of normal human serum (µl) | Concentration of E97D_del18 in the system (mg/ml) |
|---|---|---|---|
| Mouse serum control group | 100 | / | / |
| Mouse serum enzymatic digestion group | 100 | / | / |
| Human serum control group | / | 100 | 0.02 |
| Human serum enzymatic digestion group | / | 100 | 0.02 |

The systems were placed at 37°C for 24 h. 20 µl of samples were mixed with an equal volume of 2× SDS reducing loading buffer, then diluted 20x with 1× SDS reducing loading buffer, and placed in water bath at 75°C for 5 min. The cleavage products were detected by SDS-PAGE.

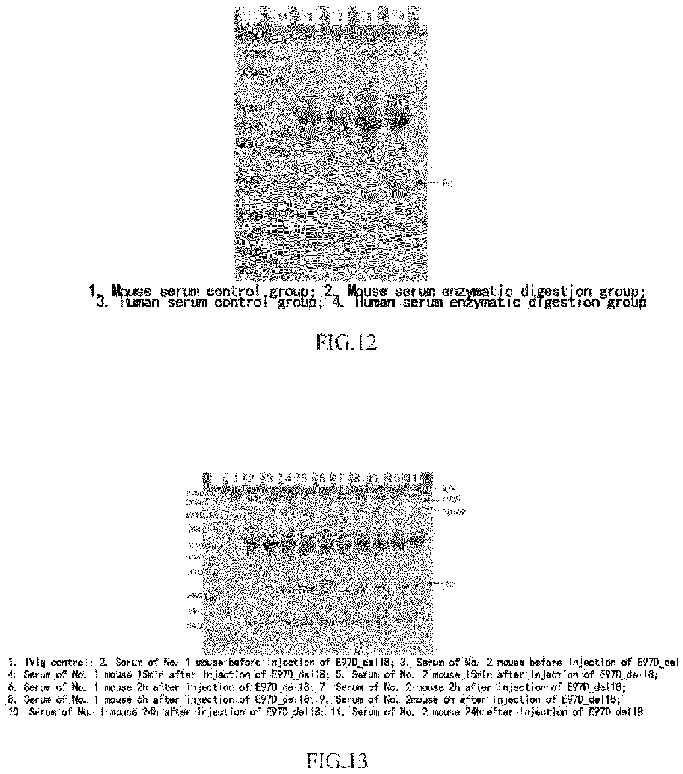

Figure 12 shows the electrophoretogram of cleavage products produced by mutant E97D_del18 in the sera of mice and humans. The results showed that an obviously visible 25 kD Fc fragment was produced in the sera of humans through E97D_del18 cleavage, which was invisible in the sera of mice, indicating that E97D_del18 can effectively and specifically cleave IgG 1 in the sera of humans, and has very low or no activity of cleaving IgG1 in the sera of mice.

### Example 8. Low pre-existing antibodies against mutant E97D del18 in humans

The assay was based on the competition between mutant E97D_del18 and IdeS for binding to anti-E97D_del18/IdeS antibodies. Pre-incubation of the test enzymes and human serum would enable the anti-E97D_del18/IdeS antibodies to bind to mutant E97D_del18 and IdeS.

A well plate was coated with mutant E97D_del18 and IdeS overnight, then washed with PBST, and blocked in 2% BSA blocking solution for 1 h. A mixed plate was prepared with the mutant to be tested and IdeS diluted stepwise and human serum. The mixed plate was incubated with shaking at room temperature for 1 h, and washed with PBST. Then, biotin-labeled E97D_del18 mutant and IdeS were added, followed by SA-HRP. The plate was developed with TMB, and read. Parallel comparison obtained the presence of pre-existing antibodies against E97D_del18 and IdeS in about eighty human blood samples.

The results are shown in Table 9, showing that the proportion of pre-existing antibodies against IdeS in normal human serum was as high as about 90%, while for mutant E97D_del18, it was only about 20%. The pre-existing antibodies against mutant E97D_del18 *in vivo* were significantly less than those against IdeS, demonstrating that mutant E97D_del18 has lower immunogenicity, and is more conducive to *in vivo* administration.

**Table 9. Comparison of pre-existing antibodies against mutant E97D_del18 and IdeS in human blood samples**

| | Mutant E97D_del18 | IdeS |
|---|---|---|
| Total number of human blood samples (No.) | 76 | 76 |
| Proportion of samples positive for pre-existing antibodies (%) | 18.4 | 89.5 |

### Example 9. In vivo detection of human IgG1-cleaving activity of mutant E97D del18

Under sterile conditions, two mice were injected with human IVIg (an intravenous human immunoglobulin) at a dose of 1 g/kg intraperitoneally (two mice were parallel experiments, numbered No. 1 and No. 2). 24 h after injecting human IVIg, the mice were injected with the IgG degrading enzyme (E97D_del18) at a dose of 5 mg/kg intravenously. 0 h, 15 min, 2 h, 6 h and 24 h after injecting E97D_del18, blood was collected from the two mice, and serum samples were collected, respectively. 20 µl of serum samples were mixed with an equal volume of 2× SDS non-reducing loading buffer, then diluted 20x with 1× SDS non-reducing loading buffer, and placed in water bath at 75°C for 5 min. The cleavage products were detected by SDS-PAGE.

Figure 13 shows the electrophoretogram of cleavage products resulting from cleavage of human IVIg by E97D_del18 at different times in the mice. The results showed that E97D_del18 effectively cleaved IVIg in the mice, and the enzymatic digestion was already substantially complete in 15 min.

### Example 10. Immunoglobulin degrading enzyme can effectively eliminate the negative influence of excess immunoglobulins on the anti-tumor effect of monoclonal antibody

A mouse model infected with Daudi cells was used to evaluate the influence of KJ103 on anti-CD38 monoclonal antibody. The experiment was designed to be four groups: 1) solvent control group; 2) anti-CD38 monoclonal antibody; 3) anti-CD38 monoclonal antibody + IVIg; and 4) anti-CD38 monoclonal antibody + immunoglobulin degrading enzyme + IVIg. Specifically, cells in the logarithmic growth phase were inoculated subcutaneously into mice, and each CB-17 SCID mouse was inoculated subcutaneously at the right armpit with 5× 10⁶ Daudi cells under sterile conditions at 0.1 mL/mouse. 1 g/kg of IVIg was dosed to the mice by single intraperitoneal injection on the day of cell inoculation. After 24 h, a single intravenous injection of 5 mg/kg of the immunoglobulin degrading enzyme E97D_del18 was dosed to the mice. Ten days after IVIg administration, 10 mg/kg of anti-CD38 monoclonal antibody was injected intraperitoneally to the mice, once a week, for a total of 4 times. After administration with the anti-CD38 monoclonal antibody, the tumor volume was measured twice a week for a total of 4 weeks.

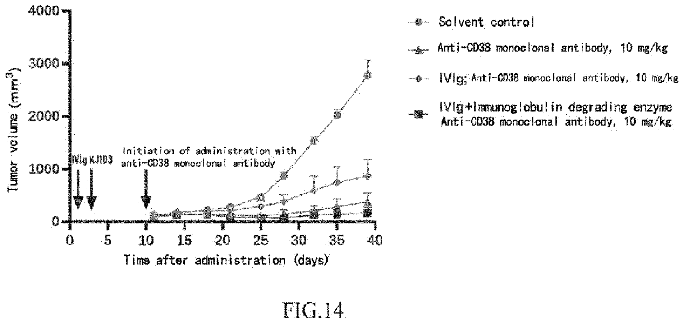

The experimental results (Figure 14) showed that the presence of IgG (IVIg) would inhibit the efficacy of anti-CD3 8 monoclonal antibody on the treatment of tumor. The immunoglobulin degrading enzyme could reduce the inhibition of the efficacy of the antibody by IgG and enhance the therapeutic effect of the therapeutic antibody.

The Applicant declares that in the present invention, the detailed methods of the present invention are illustrated by means of the above-mentioned examples, but the present invention is not limited to the detailed methods mentioned above, that is, it does not mean that the present invention must rely on the detailed methods mentioned above to carry out. Those skilled in the art should understand that any improvement of the present invention, the equivalent replacement of each raw material of the articles of manufacture of the present invention, the addition of auxiliary components, the selection of specific methods, etc., all fall within the scope of protection and the scope of disclosure of the present invention.

## Claims

1. A pharmaceutical combination comprising an immunoglobulin degrading enzyme and an Fc-containing protein, for use in the preparation of a medicament for treating hyperglobulinemia.

2. The pharmaceutical combination according to claim 1, wherein the immunoglobulin degrading enzyme is selected from an IgG cysteine protease derived from *Streptococcus pyogenes* or a variant thereof, and a human-derived MMP protease or a variant thereof, wherein the variants have at least IgG-digesting activity; and preferably, the immunoglobulin degrading enzyme is selected from one or more of IdeS, MAC2, IdeZ, IdeZ2, IdeE, IdeE2, IdeP, and MMPs.

3. The pharmaceutical combination according to claim 1 or 2, wherein the immunoglobulin degrading enzyme comprises or consists of an amino acid sequence as set forth in SEQ ID NO: 1-42, 59, 60, or 61-95.

4. The pharmaceutical combination according to any one of claims 1 to 3, wherein the Fc-containing protein is selected from one or more of an antibody, an immunoglobulin, an immunoadhesin, a therapeutic agent, a diagnostic agent, an imaging agent, and an antibody-drug conjugate.

5. The pharmaceutical combination according to any one of claims 1 to 4, further comprising one or more of a chemotherapeutic agent, a hormone agent, a proteasome inhibitor, a small molecule targeted preparation, a B-cell depleting agent, and an immunosuppressant.

6. The pharmaceutical combination according to claim 5, wherein the B-cell depleting agent is an antibody that specifically binds to CD10, CD19, CD20, CD21, CD22, CD23, CD24, CD37, CD53, CD70, CD72, CD74, CD75, CD77, CD79a, CD79b, CD80, CD81, CD82, CD83, CD84, CD85, or CD86.

7. The pharmaceutical combination according to any one of claims 1 to 6, wherein the Fc-containing protein is an antibody against one selected from CD3, CD11a, CD14, CD25, CD28, CD30, CD33, CD36, CD38, CD40, CD44, CD52, CD55, CD59, CD56, CD103, CD134, CD137, CD138, CD152, CD3, IL-1β, IL-2R, IL-6, IL-12, IL-23, C5, BAFF, BLyS, BCMA, CXCR-4, ICAM-1, SLAMF7/CS1, TNFα, and IgE.

8. The pharmaceutical combination according to claim 7, comprising an antibody that specifically recognizes CD38, wherein the antibody has binding specificity to a human effector cell, and/or the antibody is able to kill a CD38-expressing cell by apoptosis, and/or antibody-dependent cell-mediated cytotoxicity, and/or complement-dependent cytotoxicity.

9. The pharmaceutical combination according to claim 8, wherein the anti-CD38 antibody is an antibody linked to a cytotoxic agent, a radioisotope, or a drug.

10. The pharmaceutical combination according to claim 8, wherein the anti-CD38 antibody has binding specificity to CD3, CD4, CD138, IL-15R, membrane- or receptor-bound TNF-α, a human Fc receptor, or membrane- or receptor-bound IL-15.

11. The pharmaceutical combination according to claim 8, wherein the anti-CD38 antibody comprises at least one heavy chain and at least one light chain, wherein the light chain comprises three continuous complementarity determining regions as set forth in the amino acid sequences of SEQ ID NO: 43, SEQ ID NO: 44, and SEQ ID NO: 45, or three continuous complementarity determining regions as set forth in the amino acid sequences of SEQ ID NO: 49, SEQ ID NO: 50, and SEQ ID NO: 51; the heavy chain comprises three continuous complementarity determining regions as set forth in the amino acid sequences of SEQ ID NO: 46, SEQ ID NO: 47, and SEQ ID NO: 48, or three continuous complementarity determining regions as set forth in the amino acid sequences of SEQ ID NO: 52, SEQ ID NO: 53, and SEQ ID NO: 54; and an epitope-binding fragment is involved and is Fab, Fab', F(ab')₂, scFv, or a disulfide-linked Fv fragment;
preferably, the light chain comprises an amino acid sequence as set forth in SEQ ID NO: 55 or SEQ ID NO: 57, and the heavy chain comprises an amino acid sequence as set forth in SEQ ID NO: 56 or SEQ ID NO: 58;
more preferably, the amino acid sequence of the light chain is as set forth in SEQ ID NO: 55, and the amino acid sequence of the heavy chain is as set forth in SEQ ID NO: 56; or the amino acid sequence of the light chain is as set forth in SEQ ID NO: 57, and the amino acid sequence of the heavy chain is as set forth in SEQ ID NO: 58.

12. A pharmaceutical composition comprising the pharmaceutical combination according to any one of claims 1 to 11 and a pharmaceutically acceptable carrier or diluent.

13. The pharmaceutical composition according to claim 12, which is a lyophilized powder or liquid.

14. A product comprising the pharmaceutical combination according to any one of claims 1 to 11 or the pharmaceutical composition according to claim 12 or 13, wherein the immunoglobulin degrading enzyme is immobilized on a surface.

15. Use of the pharmaceutical combination according to any one of claims 1 to 11 or the pharmaceutical composition according to claim 12 or 13 in the preparation of a medicament for treating hyperglobulinemia, wherein preferably, excess globulins are produced by leukocytes selected from B cells, abnormal B cells, IgM-producing cells, IgE-producing cells, and IgA-producing cells; the globulins comprise γ-globulins; and the hyperglobulinemia comprises primary monoclonal gammaglobulinemia, malignant monoclonal gammaglobulinemia, secondary monoclonal gammaglobulinemia, leukemia, lymphoma, connective tissue disease, liver disease, infectious disease, sarcoidosis, myasthenia gravis, Hodgkin's disease, monocytic leukemia, Behcet's disease, nephritis, anaphylactoid purpura, and immune (or spontaneous) thrombocytopenia.

16. Use of the pharmaceutical combination according to any one of claims 1 to 11 or the pharmaceutical composition according to claim 12 or 13 in the preparation of a medicament for treating a CD38-related cancer or autoimmune disease, wherein the CD38-related cancer is multiple myeloma and the CD38-related autoimmune disease is systemic lupus erythematosus.

17. Use of the pharmaceutical combination according to any one of claims 1 to 11 or the pharmaceutical composition according to claim 12 or 13 in the preparation of a medicament for inhibiting the growth and/or proliferation of CD38-expressing cells.

18. Use of the pharmaceutical combination according to any one of claims 1 to 11 or the pharmaceutical composition according to claim 12 or 13 in the preparation of a medicament for treating a disease or condition in which CD38-expressing cells are involved in a patient.

19. Use of the pharmaceutical combination according to any one of claims 1 to 11 or the pharmaceutical composition according to claim 12 or 13 in the preparation of a medicament for preventing a disease or condition in which CD38-expressing cells are involved in a patient.

20. A kit comprising the pharmaceutical combination according to any one of claims 1 to 11 or the pharmaceutical composition according to claim 12 or 13.
